# EUROPEAN PATENT APPLICATION

(11) **EP 4 830 772 A1**
(43) Date of publication of application: **28.10.2026**
(21) Application number: 24907503.7
(22) Date of filing: 19.12.2024
(51) Int. Cl.: A61N 5/067

(54) **LIGHT RADIATION DEVICE**

(30) Priority: 22.12.2023 JP 2023217446; 22.12.2023 JP 2023217447
(71) Applicant: NICHIA CORPORATION, Anan-shi, Tokushima 774-8601 (JP); Illumi Medical Inc., Owariasahi-shi, Aichi 488-0061 (JP)
(72) Inventor: MIYATA, Tadaaki, Anan-shi, Tokushima 774-8601 (JP); TSUKAMOTO, Toshihiko, Owariasahi-shi, Aichi 488-0061 (JP); ENOMOTO, Kiyoshi, Anan-shi, Tokushima 774-8601 (JP)
(74) Representative: Betten & Resch
(86) International application number: PCT/JP2024/044980
(87) International publication number: WO 2025/135119

(57) **Abstract**

A light irradiation device that can indicate an irradiation direction of light is provided. The light irradiation device extending in a longitudinal direction includes a light-emitting part, and a marker member directly or indirectly connected to the light-emitting part and having radiopacity. The marker member differs in at least one of shape or position when viewed in a first direction orthogonal to the longitudinal direction and when viewed in a second direction different from the first direction and orthogonal to the longitudinal direction. The light irradiation device is configured to irradiate light from the light-emitting part in a predetermined direction that intersects the longitudinal direction.

## Description

### TECHNICAL FIELD

The present disclosure relates to a light irradiation device.

### BACKGROUND ART

For example, Patent Document 1 discloses a light irradiation device that includes a marker having radiopacity and can selectively irradiate a specific position in a biological lumen with light.

### RELATED ART DOCUMENTS

### PATENT DOCUMENT

Patent Document 1: Japanese Unexamined Patent Application Publication No. 2020-185259

### SUMMARY OF THE INVENTION

### PROBLEMS TO BE SOLVED BY THE INVENTION

It is an object of the present disclosure to provide a light irradiation device that can indicate an irradiation direction of light.

### MEANS FOR SOLVING THE PROBLEMS

In one embodiment, a light irradiation device extending in a longitudinal direction includes a light-emitting part, and a marker member directly or indirectly connected to the light-emitting part and having radiopacity. The marker member differs in at least one of shape or position when viewed in a first direction orthogonal to the longitudinal direction and when viewed in a second direction different from the first direction and orthogonal to the longitudinal direction. The light irradiation device is configured to irradiate light from the light-emitting part in a predetermined direction that intersects the longitudinal direction.

In one embodiment, a light irradiation device extending in a longitudinal direction includes a light-emitting part, a support body to which the light-emitting part is fixed, and a marker having radiopacity. The marker differs in at least one of shape or position when viewed in a first direction orthogonal to the longitudinal direction and when viewed in a second direction different from the first direction and orthogonal to the longitudinal direction. The marker is provided on the support body. The light irradiation device irradiates light from the light-emitting part in a predetermined direction that intersects the longitudinal direction.

### EFFECTS OF THE INVENTION

It is possible to provide a light irradiation device that can indicate an irradiation direction of light.

### BRIEF DESCRIPTION OF THE DRAWINGS

[FIG. 1] FIG. 1 is a schematic perspective view illustrating a configuration example of a light irradiation device according to a first embodiment.
[FIG. 2A] FIG. 2A is a schematic cross-sectional view illustrating a first example of a configuration of the light irradiation device according to the first embodiment.
[FIG. 2B] FIG. 2B is a schematic cross-sectional view illustrating a second example of the configuration of the light irradiation device according to the first embodiment.
[FIG. 3A] FIG. 3A is a diagram illustrating a marker member included in the light irradiation device according to the first embodiment, as viewed in a first direction.
[FIG. 3B] FIG. 3B is a diagram illustrating a marker member included in the light irradiation device according to the first embodiment, as viewed in a second direction.
[FIG. 4A] FIG. 4A is a schematic cross-sectional view illustrating an example of a configuration of a light irradiation device according to the second embodiment.
[FIG. 4B] FIG. 4B is a schematic bottom view illustrating the example of the configuration of the light irradiation device according to the second embodiment.
[FIG. 5] FIG. 5 is a schematic cross-sectional view illustrating a state in which the light irradiation device is rotated 180 degrees from the state illustrated in FIG. 4A with the central axis of the light irradiation device parallel to the longitudinal direction serving as the axis of rotation.
[FIG. 6] FIG. 6 is a schematic cross-sectional view illustrating an example of a configuration of a light irradiation device according to a third embodiment.
[FIG. 7] FIG. 7 is a schematic cross-sectional view illustrating an example of a configuration of a light irradiation device according to a fourth embodiment.
[FIG. 8A] FIG. 8A is a schematic cross-sectional view illustrating an example of a configuration of a light irradiation device according to a fifth embodiment.
[FIG. 8B] FIG. 8B is a schematic bottom view illustrating an example of the configuration of the light irradiation device according to the fifth embodiment.
[FIG. 9] FIG. 9 is a schematic cross-sectional view illustrating an example of a configuration of a light irradiation device according to a sixth embodiment.
[FIG. 10] FIG. 10 is a schematic perspective view illustrating an example of a configuration of a light irradiation device according to a seventh embodiment.
[FIG. 11] FIG. 11 is a diagram illustrating an example of an in vivo light irradiation assembly using a light irradiation device according to an eighth embodiment.
[FIG. 12A] FIG. 12A is a schematic view illustrating an example of a configuration of a light irradiation device according to an eighth embodiment.
[FIG. 12B] FIG. 12B is a schematic view illustrating another example of the configuration of the light irradiation device according to the eighth embodiment.
[FIG. 13] FIG. 13 is a perspective view of a main body of a light irradiation device before being sealed with an insulating layer.
[FIG. 14] FIG. 14 is a schematic view of a light-emitting element mounted on a support body.
[FIG. 15] FIG. 15 is a schematic view illustrating an example of stacking a support body and a light-emitting element.
[FIG. 16] FIG. 16 is a diagram illustrating an example of an in vivo light irradiation assembly using a light irradiation device according to a ninth embodiment.
[FIG. 17] FIG. 17 is a diagram illustrating an example of arrangement of optical elements of the light irradiation device according to the ninth embodiment.
[FIG. 18] FIG. 18 is a schematic view of a light irradiation device according to a tenth embodiment:
   [FIG. 19] FIG. 19 is a schematic view illustrating an example of a light-emitting element used in the tenth embodiment.
   [FIG. 20A] FIG. 20A is a perspective view of a light-emitting element mounting surface side of a support body used in the tenth embodiment.
   [FIG. 20B] FIG. 20B is a perspective view of a rear surface side of the support body used in the tenth embodiment.
   [FIG. 21] FIG. 21 is a graph illustrating electrical characteristics and light output characteristics of a light irradiation device of an embodiment.
   [FIG. 22] FIG. 22 is a graph illustrating thermal resistance of a light irradiation device of an embodiment.
   [FIG. 23] FIG. 23 is a schematic cross-sectional view illustrating a first example of a configuration of a light irradiation device of an eleventh embodiment.
   [FIG. 24] FIG. 24 is a schematic cross-sectional view illustrating a second example of the configuration of the light irradiation device of the eleventh embodiment.
   [FIG. 25] FIG. 25 is a schematic cross-sectional view illustrating a third example of the configuration of the light irradiation device of the eleventh embodiment.

### DESCRIPTION OF EMBODIMENTS

Embodiments for carrying out the present disclosure will be described below with reference to the drawings. The following description is intended to embody the technical concept of the present disclosure and is not intended to limit the present disclosure to the following description unless otherwise specified. In each of the drawings, members having the same function may be denoted by the same reference numerals. For convenience of explanation or ease of understanding of the main points, configurations may be described in separate embodiments; however, partial replacement or combination of configurations described in different embodiments or examples can be made. In the embodiments described later, matters different from the embodiments described earlier will be mainly explained, and redundant descriptions of matters common to the embodiments described earlier may be omitted. The sizes and positional relationships of members illustrated in each of the drawings may be exaggerated for clarity of description.

### (First Embodiment)

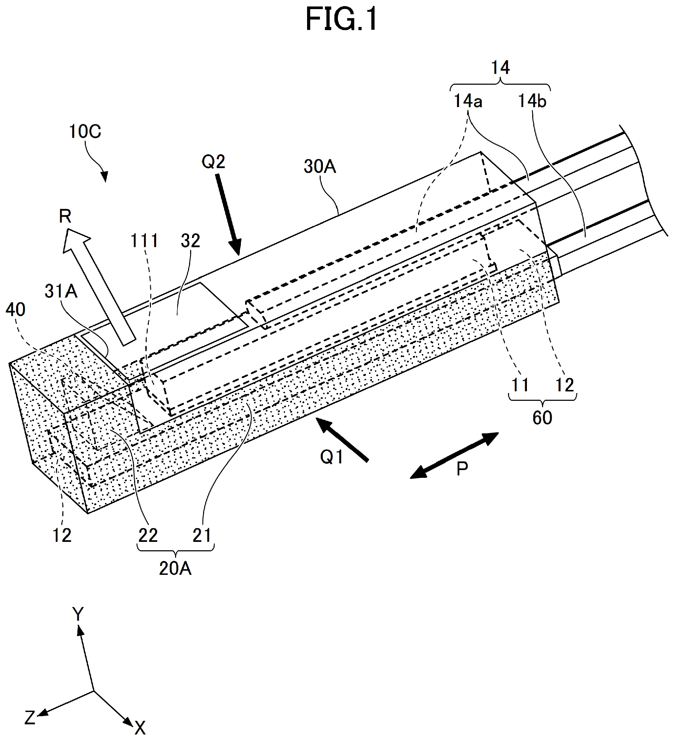

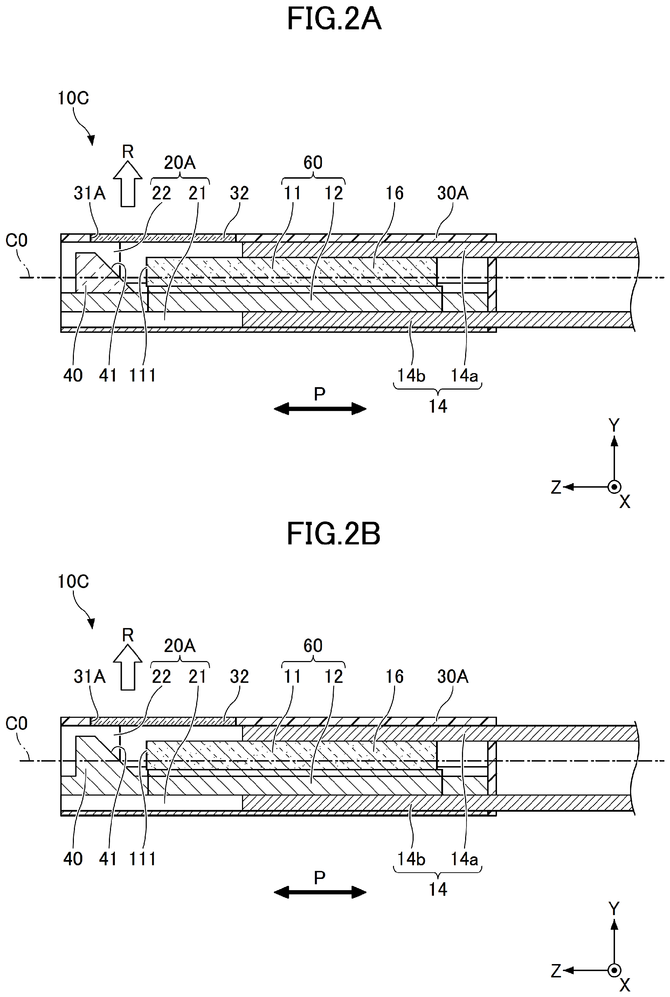

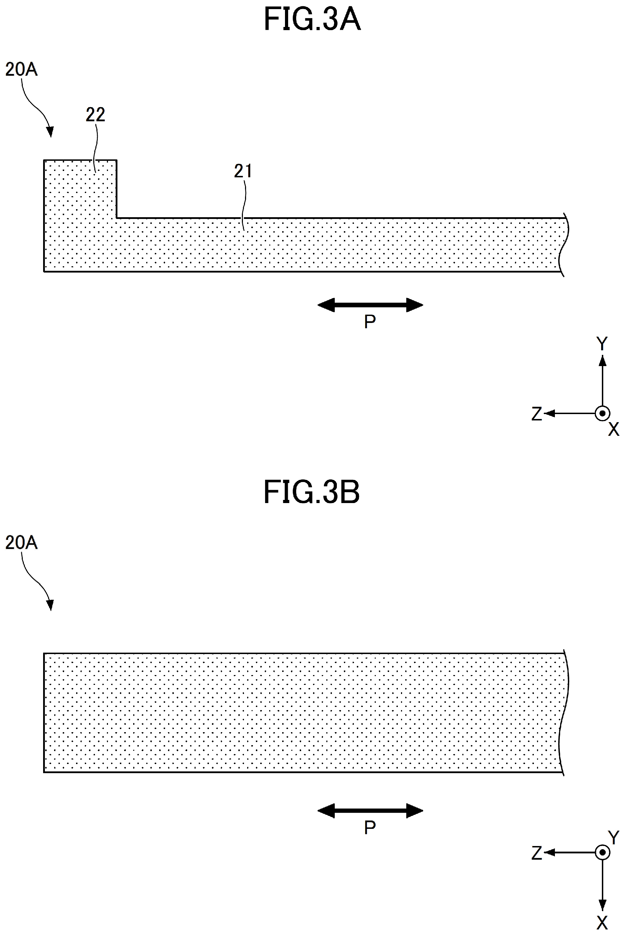

A configuration of a light irradiation device according to a first embodiment will be described with reference to FIGS. 1, 2A, 2B, 3A, and 3B. A light irradiation device 10C is, for example, inserted into a catheter and introduced into a living body. The light irradiation device 10C is inserted into the vicinity of a target site such as an affected part in a living body by using a catheter, and thus can directly irradiate an affected part, such as a tumor, with light or can be used for inspecting or confirming a target site for diagnosis or sensing with the light irradiation device 10C. FIG. 1 is a schematic perspective view illustrating an example of the configuration of the light irradiation device 10C according to the first embodiment. FIG. 2A is a schematic cross-sectional view illustrating a first example of the configuration of the light irradiation device 10C. FIG. 2B is a schematic cross-sectional view illustrating a second example of the configuration of the light irradiation device 10C. FIGS. 2A and 2B illustrate cross sections including a central axis C0 along a longitudinal direction P in which the light irradiation device 10C extends. FIG. 3A is a diagram illustrating a marker member 20A included in the light irradiation device 10C illustrated in FIGS. 1 and 2A, as viewed in a first direction Q1. FIG. 3B is a diagram illustrating a marker member 20A included in the light irradiation device 10C illustrated in FIGS. 1 and 2A, as viewed in a second direction Q2.

The central axis C0 is an axis parallel to the longitudinal direction P and passing through a center of the cross section of the light irradiation device 10C, the cross section being taken in a plane orthogonal to the longitudinal direction P. Herein, the "center" refers to the center of the circumscribed circle when the light irradiation device 10C is viewed from the +Z direction side. In the example illustrated in FIG. 2A, the light irradiation device 10C has a substantially rectangular-parallelepiped outer shape elongated in the longitudinal direction P, in which a casing 30A and the marker member 20A are coupled. In the example illustrated in FIG. 2A, the cross section of the light irradiation device 10C orthogonal to the axis parallel to the longitudinal direction P has a substantially rectangular shape. In the example illustrated in FIG. 2A, the center of the cross section of the light irradiation device 10C orthogonal to the axis parallel to the longitudinal direction P corresponds to the center of the cross-section of the rectangular parallelepiped shape orthogonal to the axis parallel to the longitudinal direction P. However, the shape of the cross section of the light irradiation device 10C orthogonal to the axis parallel to the longitudinal direction P is not limited to a substantially rectangular shape, and may be any shape. The center of the cross section of the light irradiation device 10C orthogonal to the axis parallel to the longitudinal direction P may be substantially the center of any shape orthogonal to the axis parallel to the longitudinal direction P.

As illustrated in FIGS. 1 and 2A, the light irradiation device 10C includes a light-emitting part 60 and the marker member 20A which is directly or indirectly connected to the light-emitting part 60 and has radiopacity. As illustrated in FIGS. 3A and 3B, the marker member 20A when viewed in the first direction Q1, which is orthogonal to the longitudinal direction P is different in at least one of shape or position from the marker member 20A when viewed in the second direction Q2, which is different from the first direction Q1 and orthogonal to the longitudinal direction P. The light irradiation device 10C can irradiate light from the light-emitting element 11 in a predetermined direction R that intersects the longitudinal direction P.

For example, in a light irradiation device for medical use, for the purpose of irradiating light onto a target part in the living body, it is sometimes required to determine an irradiation direction of light from the light irradiation device in a state in which the light irradiation device is positioned in a living body. The light irradiation device 10C is radiopaque to radiation such as X-rays, and includes the marker member 20A of which at least one of shape or position is different between when viewed in the first direction Q1 and when viewed in the second direction Q2. Components other than the marker member 20A in the light irradiation device 10C are radiolucent to radiation such as X-rays. Accordingly, when a living body in which the light irradiation device 10C is positioned is imaged by X-ray CT (computed tomography) or the like, an image of the marker member 20A can be confirmed in the captured image. By confirming at least one of shape or position of the image of the marker member 20A appearing in the captured image by X-ray CT, the orientation of the marker member 20A arranged in the living body can be identified. The orientation of the marker member 20A is known with respect to the longitudinal direction P, and the light irradiation device 10C can irradiate light from the light-emitting element 11 in the predetermined direction R that intersects the longitudinal direction P. Therefore, in the present embodiment, in using the light irradiation device 10C, it is possible to determine the irradiation direction of light from the light irradiation device 10C based on the orientation of the marker member 20A, which is checked on the basis of the captured image by X-ray CT. In another viewpoint, the inclusion of the marker member 20A enables the irradiation direction of light from the light irradiation device 10C to be identified by X-ray CT or the like. According to the present embodiment, the light irradiation device 10C that can indicate an irradiation direction of light can be provided.

Herein, the phrase "at least one of shape or position is different" includes a case in which the difference in shape and/or position is visually observable, and does not necessarily indicate that the difference in shape and/or position appears in a radiographic image. In the radiographic image, the shape and the position of the marker member 20A illustrated in FIGS. 1 and 2A when viewed from above (for example, the +Y side) are the same as those when viewed from below (for example, the -Y side). Therefore, the irradiation direction of light from the light irradiation device 10C cannot be determined from this image alone. However, because the marker member 20A has a structure in which at least one of its shape or position differs when visually observed, a slight change in viewing direction results in change in appearance of the radiographic image, thereby allowing determination of whether the light is irradiated from above or from below.

The marker member 20A illustrated in FIGS. 3A and 3B has an elongated base portion 21 and a protruding portion 22 provided on the surface of the base portion 21. Thus, the marker member 20A having a shape different between when viewed in the first direction Q1 and when viewed in the second direction Q2 can be provided. The marker member 20A can contain a metal material having radiopacity to radiation, such as platinum.

The marker member 20A is not limited to a configuration having the protruding portion 22, and may be provided with at least one of a protrusion or a recess on the surface of the base portion 21. Furthermore, the marker member 20A is not limited to a configuration having the base portion 21 and at least one of a protrusion or a recess, but may have various shapes as long as at least one of shape or position differs between when viewed in the first direction Q1 and when viewed in the second direction Q2. One or more portions of the marker member 20A may be arranged so as to be separated from other portions.

In the examples illustrated in FIGS. 1 and 2A, the light-emitting part 60 has the light-emitting element 11 and a support body 12 for supporting the light-emitting element 11. The support body 12 is arranged on the upper surface (for example, on the +Y side) of the base portion 21 of the marker member 20A. The light-emitting element 11 is arranged on the upper surface of the support body 12. The light-emitting element 11 can emit light in a direction along the longitudinal direction P from a light-emitting surface 111 that intersects the longitudinal direction P. In the example illustrated in FIG. 1, the light-emitting element 11 emits light in the +Z direction. With the light-emitting part 60 including the light-emitting element 11 and the support body 12, the support body 12 can be used as a heat dissipating member for dissipating heat of the light-emitting element 11 in the light irradiation device 10C.

In the examples illustrated in FIGS. 1 and 2A, the light irradiation device 10C includes an optical component 40 that causes light emitted from the light-emitting element 11 in a direction along the longitudinal direction P to travel in the direction R that intersects the longitudinal direction P. In the examples illustrated in FIGS. 1 and 2A, the optical component 40 is a prism that is arranged on the upper surface of the support body 12 and includes a reflecting surface 41 that intersects the upper surface of the support body 12. Light emitted from the light-emitting element 11 is reflected at the reflecting surface 41, so that the optical component 40 causes the light to travel in the direction R. The optical component 40 is not limited to a prism and may be a mirror, a lens, a diffractive optical element, or the like as long as it is possible to direct light in a predetermined direction. The optical component 40 may include a resin material, a glass material, a metal material, or the like. As illustrated in FIG. 2B, the optical component 40 may be integrally formed with the support body 12.

In the example illustrated in FIG. 2A, the insulating layer 16 covers the entire surface of the light-emitting element 11. This prevents damage to the light-emitting element 11 due to short-circuiting between the marker member 20A and the light-emitting element 11 via the support body 12. In the light irradiation device 10C, the insulating layer 16 is not necessarily limited to cover the entire surface of the light-emitting element 11 but may cover at least one of the light-emitting element 11 and the support body 12. With the insulating layer 16 covering at least one of the light-emitting element 11 or the support body 12, it is possible to prevent breakage of the light-emitting element 11 due to short-circuiting between the marker member 20A and the light-emitting element 11.

In the examples illustrated in FIGS. 1 and 2A, the light irradiation device 10C includes insulated wires 14 electrically connected to the light-emitting element 11, and the insulated wires 14 are electrically insulated from the marker member 20A. As described above, the insulated wires 14 include a first insulated wire 14a and a second insulated wire 14b. With the light irradiation device 10C including the insulated wires 14 electrically connected to the light-emitting element 11, it is possible to prevent breakage of the light-emitting element 11 due to short-circuiting via the marker member 20A.

In the examples illustrated in FIGS. 1 and 2A, the light irradiation device 10C has the casing 30A including an opening 31A and configured to accommodate at least a part of the light-emitting element 11 and at least a part of the support body 12, and a light-transmissive member 32 that seals the opening 31A of the casing 30A. The light-transmissive member 32 transmits light emitted from the light-emitting part 60 and irradiated in the predetermined direction R that intersects the longitudinal direction P. With this configuration, the light irradiation device 10C can irradiate light emitted from the light-emitting element 11 arranged inside the casing 30A through the light-transmissive member 32. Sealing the opening 31A with the light-transmissive member 32 allows for inhibiting the inside of the casing 30A from being exposed to the outside. The casing 30A can contain a resin material or a metal material or the like having light shielding property or absorption property with respect to the peak wavelength of light emitted from the light-emitting element 11. The light-transmissive member 32 can include a resin material or a glass material or the like having transmissivity with respect to the peak wavelength of light emitted from the light-emitting element 11.

In the examples illustrated in FIGS. 1 and 2A, the light-emitting element 11 is arranged inside the casing 30A, and the inside of the casing 30A is hermetically sealed. Thus, even when the light irradiation device 10C is arranged in a biological lumen or the like where a liquid such as blood exists, the liquid can be prevented from entering the inside of the casing 30A, and the light-emitting element can be prevented from being damaged by a short circuit caused by the liquid.

The materials of the support body 12, the light-emitting element 11, the optical component 40, the insulated wires 14 and the like can be the same as those of the eighth through tenth embodiments described later, and the same applies to the second through seventh embodiments. As in the example illustrated in FIG. 2B, when the support body 12 and the optical component 40 are integrally formed, they may be integrally formed of the same material. In view of having a high thermal conductivity, aluminum nitride (AlN) may be included as a main component. As an example of a method for integrally forming the support body 12 and the optical component 40, such an integrated component can be formed by injection molding or pressing a ceramic green sheet.

### (Second Embodiment)

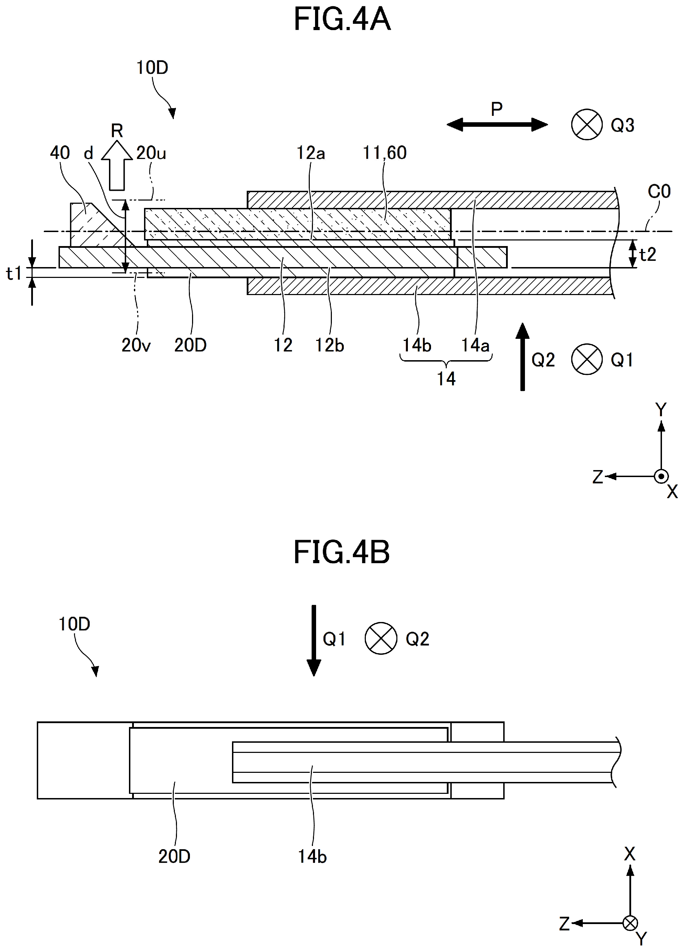

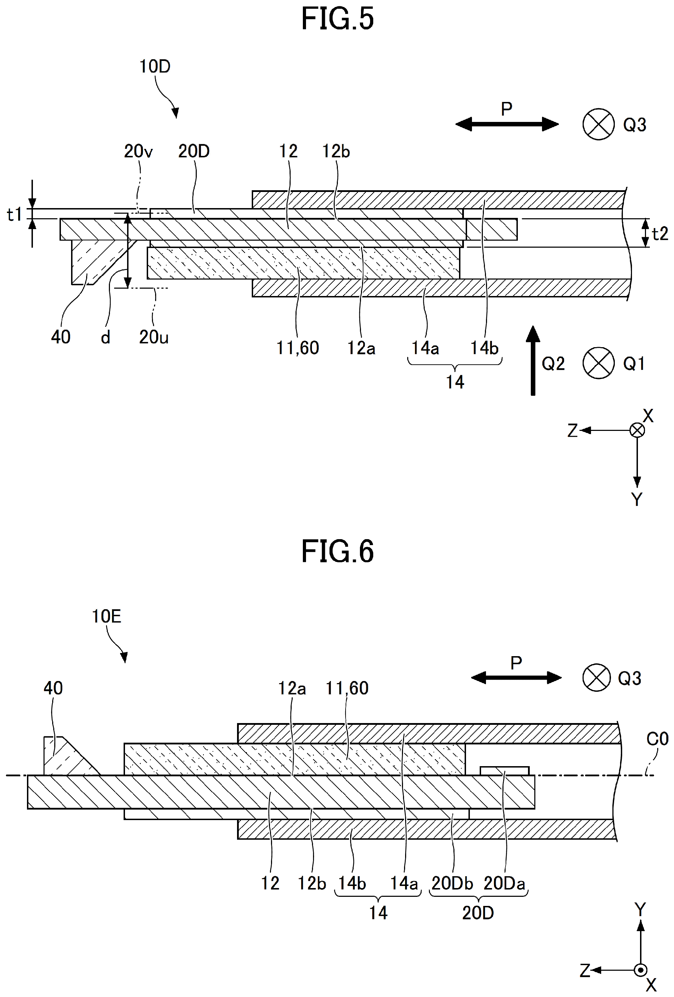

The configuration of a light irradiation device according to the second embodiment will be described with reference to FIGS. 4A, 4B, and 5. FIG. 4A is a schematic cross-sectional view illustrating an example of the configuration of a light irradiation device 10D according to the second embodiment. FIG. 4B is a schematic bottom view illustrating an example of the configuration of the light irradiation device 10D. FIG. 5 is a schematic cross-sectional view illustrating a state in which the light irradiation device 10D is rotated 180 degrees from the state illustrated in FIG. 4A with the central axis C0 of the light irradiation device 10D parallel to the longitudinal direction P serving as a rotation axis. FIGS. 4A and 5 illustrate cross sections including the central axis C0 along the longitudinal direction P in which the light irradiation device 10D extends.

As illustrated in FIGS. 4A, 4B, and 5, the light irradiation device 10D includes the light-emitting element 11, the support body 12 to which the light-emitting element 11 is fixed, and a marker 20D having radiopacity. At least one of shape or position of the marker 20D differs between when viewed in the first direction Q1, which is orthogonal to the longitudinal direction P, and when viewed in the second direction Q2, which is different from the first direction Q1 and orthogonal to the longitudinal direction P. The marker 20D is provided on the support body 12. The light irradiation device 10D can irradiate light from the light-emitting element 11 in the predetermined direction R that intersects the longitudinal direction. In the present embodiment, the light-emitting element 11 is an example of the light-emitting part 60. In FIG. 4A, the reference numerals for the light-emitting element 11 and the light-emitting part 60 are shown together to indicate that they refer to the same component. In the following drawings, reference numerals may similarly be presented together for the same purpose.

In the present embodiment, the inclusion of the marker 20D enables the irradiation direction of light from the light irradiation device 10D to be identified by X-ray CT or the like, as in the first embodiment described above. This allows for providing the light irradiation device 10D that can indicate an irradiation direction of light. In the present embodiment, the marker 20D is provided on the support body 12, allowing simplification of the configuration of the light irradiation device as compared with a case where a member functioning as a marker is provided other than the support body 12. The marker 20D is fixed to the support body 12 and can move integrally with the support body 12. Also in the present embodiment, the phrase "at least one of shape or position is different" includes a case in which the difference in shape and/or position is visually observable, and does not necessarily indicate that the difference in shape and/or position appears in a radiographic image. In the radiographic image, the shape and the position of the marker 20D illustrated in FIGS. 4A, 4B, and 5 when viewed from above (for example, the +Y side) are the same as those when viewed from below (for example, the -Y side). Therefore, the irradiation direction of light from the light irradiation device 10D cannot be identified from this image alone. However, because the marker 20D has a structure in which at least one of its shape or position differs when visually observed, a slight change in viewing direction results in change in appearance of the radiographic image, thereby allowing determination of whether the light is irradiated from above or from below.

In the example illustrated in FIGS. 4A, 4B, and 5, the support body 12 includes a first surface 12a on which the light-emitting element 11 is arranged, and a second surface 12b located on the side opposite to the first surface 12a. The marker 20D is provided on the second surface 12b of the support body 12. With this configuration, in the light irradiation device 10D, the region on the support body 12 can be effectively utilized by providing the marker 20D on the second surface 12b of the support body 12 on the side opposite to the first surface 12a on which the light-emitting element 11 is arranged. The marker 20D of the first surface 12a may have anisotropy when viewed in a direction parallel or orthogonal to the first surface 12a, and the marker 20D of the second surface 12b may have anisotropy in a direction different from that of the first surface 12a. The term "anisotropy of the marker 20D" herein means a characteristic in which at least one of shape or position of the marker 20D is varied.

In the light irradiation device 10D, the marker 20D includes a metal material, and the thickness of at least a part of the marker 20D is equal to or greater than 20 µm and equal to or less than 100 µm. In the example illustrated in FIGS. 4A, 4B, and 5, the marker 20D is a plate-like member having a thickness t1. The thickness t1 is equal to or greater than 20 µm and equal to or less than 100 µm. It is preferable that at least a portion of the marker 20D have a thickness of 40 µm or greater, because this improves visibility in a radiographic image. It is possible to reduce the size when the thickness is 100 µm or less.

Platinum or the like can be used as a metal material constituting the marker 20D. The marker 20D is provided by plating platinum on the second surface 12b of the support body 12. However, the marker 20D is not limited to a portion provided on another component, such as the support body 12. The marker 20D may be a component that is joined to another component such as the support body 12 by an adhesive component or the like and separate from the other components.

In the light irradiation device 10D, the position of the marker 20D when viewed in a third direction Q3, which is a direction parallel to the first surface 12a, differs from the position of the marker 20D when viewed in the third direction Q3 by rotating the light irradiation device 10D by 180 degrees with the central axis C0 of the light irradiation device 10D parallel to the longitudinal direction P as a rotation axis by at least twice a distance t2 between the first surface 12a and the second surface 12b in the support body 12. A first position 20v illustrated in FIG. 4A represents the position of the marker 20D when viewed in the third direction Q3, which is a direction parallel to the first surface 12a. On the other hand, a second position 20u illustrated in FIG. 5 represents the position of the marker 20D when viewed in the third direction Q3 by rotating the light irradiation device 10D by 180 degrees with the central axis C0 of the light irradiation device 10D parallel to the longitudinal direction P as a rotation axis. The second position 20u illustrated in FIG. 4A virtually represents the second position 20u in the state illustrated in FIG. 5 for comparison with the first position 20v. The distance d between the first position 20v and the second position 20u is twice or more of the distance t2. This configuration can facilitate recognition of the difference in the position of the marker 20D according to the viewing direction. In the present embodiment, differences in the position of the marker 20D depending on the viewing direction can be easily recognized, which allows the direction of light irradiated from the light irradiation device 10D to be clearly identified by X-ray CT or the like through the use of the marker 20D.

In the light irradiation device 10D, the light-emitting part is the light-emitting element 11. The light-emitting element 11 is arranged on the support body 12. With this configuration, the light irradiation device 10D can be provided with a light source, so that the position of the light irradiation device 10D can be easily controlled.

In the example illustrated in FIGS. 4A, 4B, and 5, the optical component 40 of the light irradiation device 10D is mounted on the support body 12.

### (Third Embodiment)

The configuration of a light irradiation device according to the third embodiment will be described with reference to FIG. 6. FIG. 6 is a schematic cross-sectional view illustrating an example of the configuration of a light irradiation device 10E according to the third embodiment. FIG. 6 illustrates a cross section including the central axis C0 along the longitudinal direction P in which the light irradiation device 10E extends.

In the light irradiation device 10E, markers 20D are provided on both of the first surface 12a and the second surface 12b. The marker 20D provided on the first surface 12a differs from the marker 20D provided on the second surface 12b in at least one of size or position. This configuration can facilitate recognition of the difference in the position of the markers 20D according to the viewing direction, so that the direction of light from the light irradiation device 10E can be clearly identified by X-ray CT or the like.

In the example illustrated in FIG. 6, the markers 20D include a first marker 20Da and a second marker 20Db. The first marker 20Da is provided on the first surface 12a side by side with the light-emitting element 11 along the longitudinal direction P. The second marker 20Db is provided on the second surface 12b. The first marker 20Da is provided at a position shifted from the second marker 20Db in the longitudinal direction P. The first marker 20Da is smaller than the second marker 20Db. In other words, the first marker 20Da differs from the second marker 20Db in size and position. Also in the present embodiment, the phrase "at least one of shape or position is different" includes a case in which the difference in shape and/or position is visually observable, and does not necessarily indicate that the difference in shape and/or position appears in a radiographic image. In the radiographic image, the shape and the position of the marker 20D illustrated in FIG. 6 when viewed from above (for example, the +Y side) are the same as those when viewed from below (for example, the -Y side). Therefore, the irradiation direction of light from the light irradiation device 10E cannot be identified from this image alone. However, because the marker 20D has a structure in which at least one of its shape or position differs when visually observed, a slight change in viewing direction results in a change in appearance of the radiographic image, thereby allowing determination of whether the light is irradiated from above or from below. The presence of two directions that differ in visual observation but does not differ in radiographic images is the same as that in the fourth through seventh embodiments, which will be described later; therefore, description of the presence of two directions will be omitted in the fourth through seventh embodiments.

### (Fourth Embodiment)

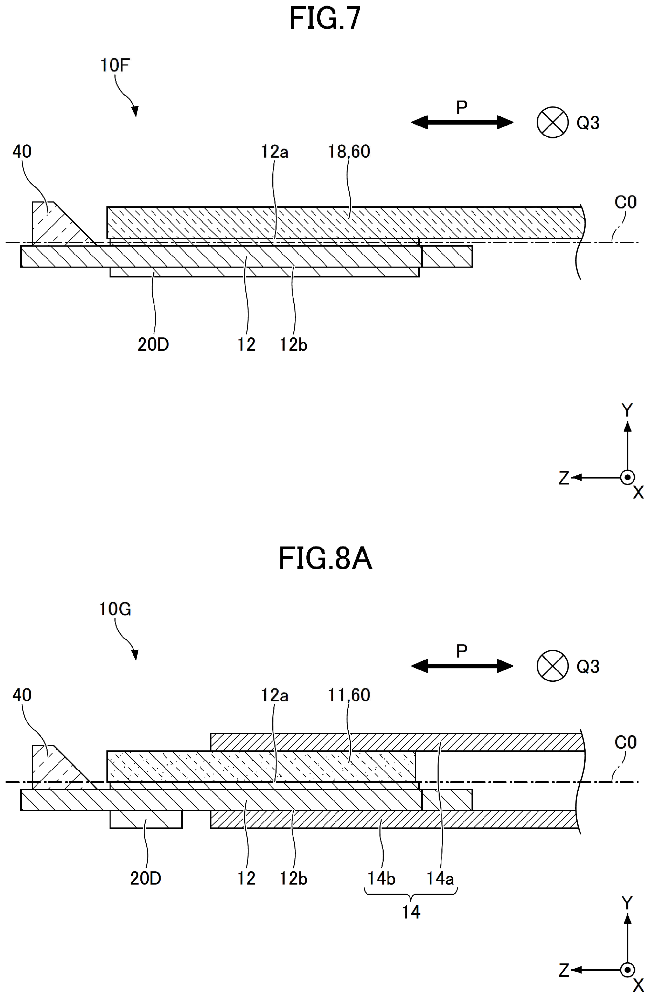

The configuration of a light irradiation device according to the fourth embodiment will be described with reference to FIG. 7. FIG. 7 is a schematic cross-sectional view illustrating an example of the configuration of a light irradiation device 10F according to the fourth embodiment. FIG. 7 illustrates a cross section including a central axis C0 along the longitudinal direction P in which the light irradiation device 10F extends.

In the light irradiation device 10F, the light-emitting part is a fiber 18. The fiber 18 is arranged on the support body 12. With this configuration, the light irradiation device 10F can guide light from a light source arranged at a location apart from it, so that the light source need not be provided inside the light irradiation device. Because the light irradiation device 10F does not include a light source, the configuration of the light-emitting part 60 of the light irradiation device 10F can be simplified.

### (Fifth Embodiment)

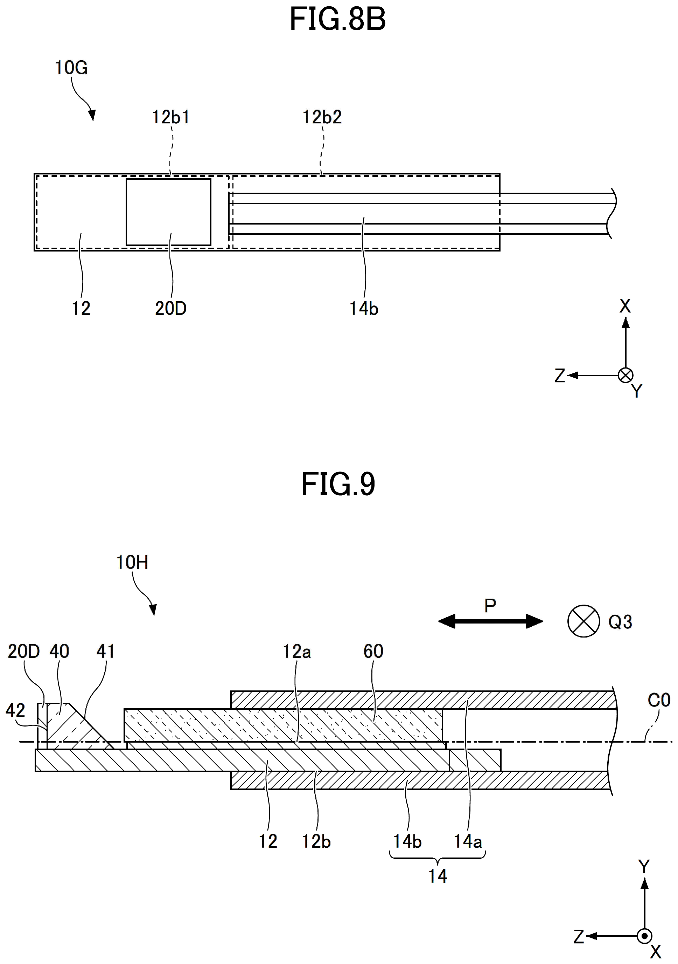

The configuration of a light irradiation device according to the fifth embodiment will be described with reference to FIGS. 8A and 8B. FIG. 8A is a schematic cross-sectional view illustrating an example of the configuration of a light irradiation device 10G according to the fifth embodiment. FIG. 8A illustrates a cross section including a central axis C0 along the longitudinal direction P in which the light irradiation device 10G extends. FIG. 8B is a schematic bottom view illustrating an example of the configuration of the light irradiation device 10G.

As illustrated in FIG. 8B, in the light irradiation device 10G, the second surface 12b of the support body 12 includes a first region 12b1 and a second region 12b2 different from the first region 12b1. A marker 20D is provided in the first region 12b1, and the insulated wire 14 is arranged in the second region 12b2. In the examples illustrated in FIGS. 8A and 8B, the second insulated wire 14b is arranged in the second region 12b2.

In the light irradiation device 10G, the marker 20D and the insulated wire 14 are arranged on the second surface 12b of the support body 12, allowing for reducing the thickness of the light irradiation device 10G as compared with the case where the insulated wire 14 is arranged on the marker 20D provided on the second surface 12b.

### (Sixth Embodiment)

The configuration of a light irradiation device according to the sixth embodiment will be described with reference to FIG. 9. FIG. 9 is a schematic cross-sectional view illustrating an example of the configuration of a light irradiation device 10H according to the sixth embodiment.FIG. 9 illustrates a cross section including a central axis C0 along the longitudinal direction P in which the light irradiation device 10H extends.

The light irradiation device 10H is mainly different from the above-described embodiments in that it includes the optical component 40 that has an optical effect of at least one of reflection, refraction, or diffraction with respect to light emitted from the light-emitting part 60, and the marker 20D is provided on the optical component 40.

In the light irradiation device 10H, the light-emitting part 60 emits light in a direction along the longitudinal direction P, and the optical component 40 includes a reflecting surface 41 which reflects light from the light-emitting part 60 in a direction that intersects the longitudinal direction P. The marker 20D is provided in a region of the optical component 40 other than the region where the reflecting surface 41 is provided. In the example illustrated inFIG. 9, the optical component 40 is arranged on the upper surface of the support body 12. The optical component 40 is a prism having a reflecting surface 41 and a surface 42 located on the opposite side of the reflecting surface 41. The marker 20D is provided on the surface 42 of the optical component 40. In the light irradiation device 10H, the configuration of the light irradiation device can be simplified by providing the marker 20D on the optical component 40. However, the marker 20D may be provided on a side surface of the optical component 40 that intersects the surface 42, for example, at least one of the +X side surface and the - X side surface of the optical component 40.

### (Seventh Embodiment)

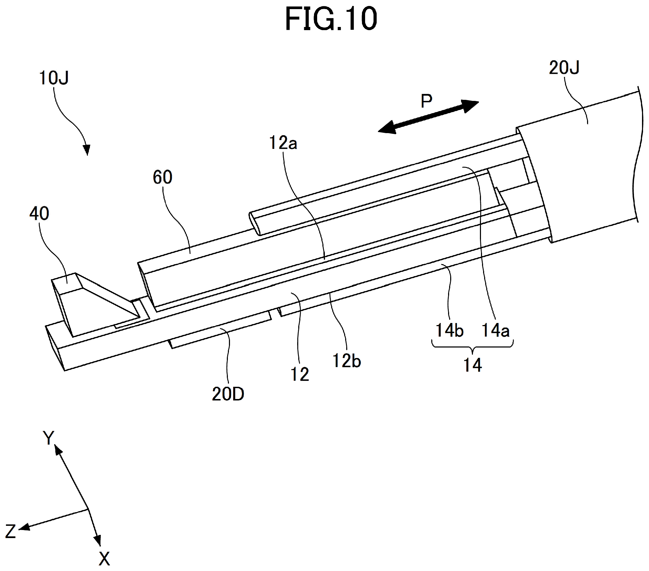

The configuration of a light irradiation device according to the seventh embodiment will be described with reference to FIG. 10. FIG. 10 is a schematic perspective view illustrating an example of the configuration of a light irradiation device 10J according to the seventh embodiment.

In addition to the marker 20D, the light irradiation device 10J includes a second marker 20J in a portion other than the support body 12 and the optical component 40. In the example illustrated in FIG. 10, the light irradiation device 10J includes the marker 20D provided on the support body 12 and the second marker 20J arranged on the opposite side of the optical component 40 with the light-emitting part 60 therebetween. The second marker 20J is a cylindrical member configured to accommodate the insulated wires 14 therein. However, the shape of the second marker 20J is not limited to a cylindrical shape and may be any shape.

In the light irradiation device 10J, by providing the second marker 20J, the portion corresponding to the second marker 20J that appears in the captured image of X-ray CT can be enlarged as compared with a case where only the marker 20D is provided. This can improve the visibility of the marker in captured images, allowing for providing the light irradiation device 10J of which position can be easily determined. On the other hand, in a case where only the marker 20D is provided without the second marker 20J, the accurate position and direction can be determined by using the marker 20D. Thus, the light irradiation device 10J that can indicate an irradiation direction of light with high accuracy can be provided.

The eighth through tenth embodiments will be described hereinafter. Although the eighth through tenth embodiments describe examples in which no marker member or marker is provided, the light irradiation device described in the eighth through tenth embodiments may be provided with the marker member or marker described in the first through seventh embodiments.

### (Eighth Embodiment)

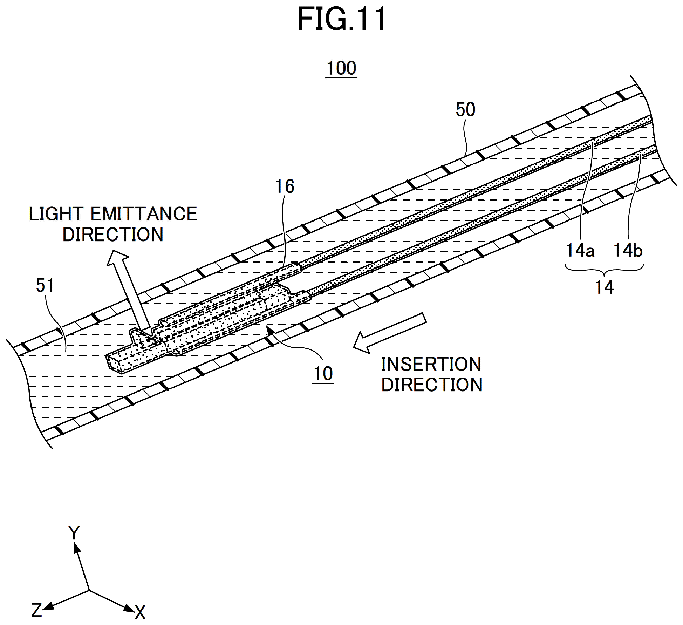

FIG. 11 is a diagram illustrating an example of an in vivo light irradiation assembly 100 using a light irradiation device 10 of the eighth embodiment. The in vivo light irradiation assembly 100 includes a catheter 50 and a light irradiation device 10 inserted into the catheter 50, and the inside of the catheter 50 is filled with a refrigerant 51 at least during use. The in vivo light irradiation assembly 100 is used for treatment, diagnosis, sensing, and the like. The light irradiation device 10 is, for example, inserted into the catheter 50 in a direction indicated by the white arrow labeled as "insertion direction" in the figure and introduced into a living body. Because the light irradiation device 10 is a heating element, the refrigerant 51 is supplied into the catheter 50 and used in a state where the heating portion is cooled. For this reason, the insulating layer 16 is integrally provided over the tip side of the light irradiation device 10. The refrigerant 51 is, for example, physiological saline, blood, lymph or the like, and the refrigerant temperature is, for example, about 10 °C to 36 °C. Because the light irradiation device 10 is inserted into the vicinity of an irradiation target site such as an affected part in a living body by using the catheter 50, it is possible to directly irradiate an affected part such as a tumor or the like with light without using an optical fiber, or to inspect or check a target site such as diagnosis or sensing.

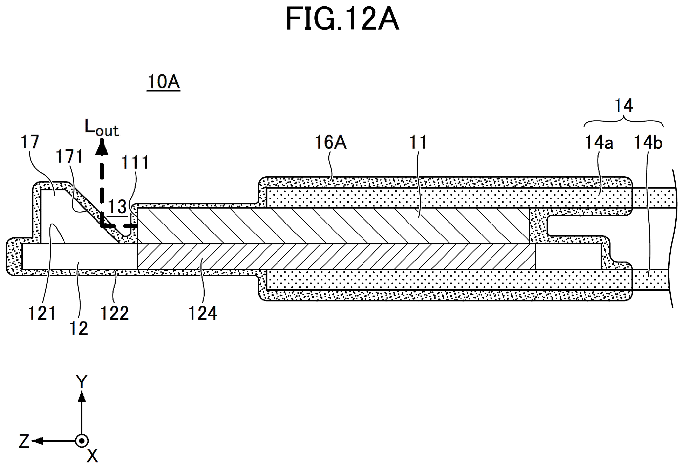

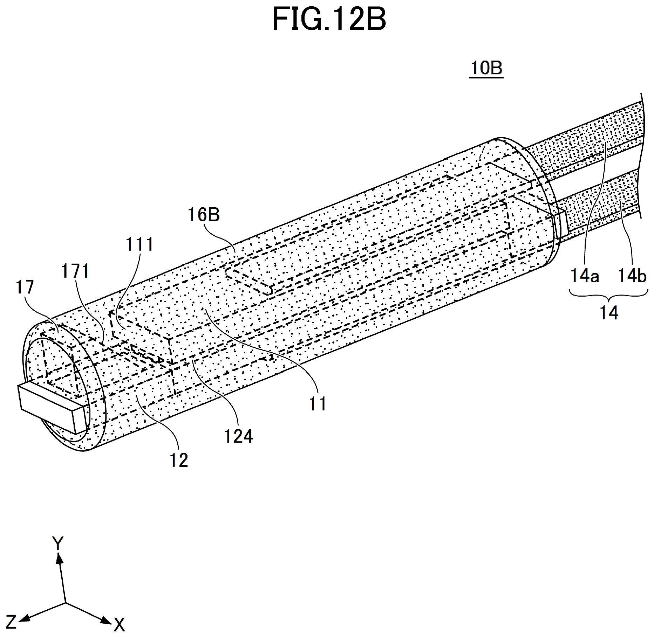

FIGS. 12A and 12B are schematic views illustrating configuration examples of the light irradiation device 10 of the eighth embodiment illustrated in FIG. 1. In each of a light irradiation device 10A of FIG. 12A and a light irradiation device 10B of FIG. 12B, a light-emitting element, a support body on which the light-emitting element is mounted, and an insulated wire electrically connected to the light-emitting element are integrally covered with an insulating layer, but the manner in which the insulating layer is provided differs between these components. In the coordinate system in each of FIGS. 12A and 12B, the optical axis direction is the Z direction, the mounting direction of the light-emitting element on the support body is the Y direction, and the direction orthogonal to the Z direction and the Y direction is the X direction. The light irradiation device 10A of FIG. 12A includes: the light-emitting element 11 configured to emit light of a predetermined wavelength; an optical component 17 on which light emitted from the light-emitting element 11 enters; the support body 12 on which the light-emitting element 11 and the optical component 17 are mounted; the insulated wires 14 electrically connected to the light-emitting element 11; and an insulating layer 16A for integrally covering the outer surface of a structure including the light-emitting element 11, the optical component 17, the support body 12, and the insulated wire 14. The insulating layer 16A is, for example, a coat (hereinafter referred to as "polysilazane coat") obtained by applying a polysilazane solution and forming into silica, and is light-transmissive to light emitted by the light-emitting element 11. By employing the polysilazane coat, it is possible to reduce the thickness of the insulating layer to enhance heat dissipation and to ensure insulation. The light-emitting surface 111 of the light-emitting element 11 and a light incident surface 171 of the optical component 17 face each other across a space 13. Light emitted by the light-emitting surface 111 enters the light incident surface 171 of the optical component 17 through the insulating layer 16A, the space 13, and the insulating layer 16A. The insulating layer 16A in FIG. 12A seals the stacked structure along the surface shape of the support body 12 and components such as the light-emitting element 11 mounted on the support body 12.

In this configuration example, the longitudinal direction of the support body 12 is parallel to the Z direction. The light irradiation device 10 of the eighth embodiment is inserted into the catheter 50 in the +Z direction, and the insulated wires 14 including the first insulated wire 14a and the second insulated wire 14b extends in the -Z direction. The support body 12 is made of an insulating material such as silicon (Si), aluminum nitride (AlN), silicon nitride (SiN), alumina (Al₂O₃), glass, quartz, or ceramics, and preferably a material having a thermal conductivity of 100 W/m·K or more. Furthermore, not only the insulating material but also a metal material such as copper or a resin material may be used. In the case where a metal material is used, an insulating treatment is appropriately performed so as not to cause a short circuit or the like. The light-emitting element 11 is mounted on a light-emitting element mounting surface 121 of the support body 12.

The optical component 17 is mounted on the light-emitting element mounting surface 121 of the support body 12 together with the light-emitting element 11, and the light incident surface 171 of the optical component 17 is inclined with respect to the light-emitting element mounting surface 121. Thus, the light incident surface 171 of the optical component 17 functions as a reflecting surface, and the output light Lₒᵤₜ of the light irradiation device 10A is extracted in the direction of the broken-line arrow in the figure. In the configuration example of FIG. 12A, the optical component 17 is a reflective member that directs light emitted by the light-emitting element 11 in a direction (for example, in the +Y direction) that intersects the light-emitting element mounting surface 121 of the support body 12. Thus, light can be emitted in the circumferential direction of the light irradiation device. The optical component 17 may be a mirror that reflects light incident on the light incident surface 171. The mirror may include a reflective layer of a metal, a dielectric multilayer film, or both. Thus, light can be reflected efficiently.

The insulated wires 14 include the first insulated wire 14a that is electrically connected to one electrode (also referred to as a "first electrode") of the light-emitting element 11 on the side of the light-emitting element mounting surface 121 that is the first surface of the support body 12 on which the light-emitting element 11 is mounted, and a second insulated wire 14b that is electrically connected to the other electrode (also referred to as a second electrode) of the light-emitting element 11 on the side of the rear surface 122 (also referred to as a "second surface") that is opposite to the light-emitting element mounting surface 121. The use of the insulated wire makes it possible to energize the light-emitting element 11. As one of the features of the light irradiation device 10A, electrical connections between the first insulated wire 14a and the second insulated wire 14b and the light-emitting element 11 are integrally sealed with an insulating layer 16A together with the support body 12, the light-emitting element 11, and the optical component 17. The insulating layer 16A has a thickness smaller than that of the light-emitting element 11. By integrally covering the light irradiation device 10A with the insulating layer 16 having the thickness smaller than that of the light-emitting element 11, the size of the light irradiation device 10A can be reduced while securing electrical insulation, thereby facilitating insertion into the catheter 50. With the insulating layer 16A thinner than the thickness of the light-emitting element, thermal resistance of the insulating layer 16 can be reduced, and heat dissipation performance can be enhanced.

Although the width of the support body 12 in the X direction is a width which can be easily inserted into the catheter 50, it may be set as wide as possible within a range which can be smoothly inserted into the catheter 50 from the viewpoint of enlarging a cooling area (i.e. heat dissipation area) by the refrigerant 51. The thickness of the support body 12 in the Y direction is a thickness which can stably support the light-emitting element 11 and can be smoothly inserted into the catheter 50 with the light-emitting element 11 mounted thereon. As an example, the thickness of the support body 12 is about 0.075 mm to 0.3 mm.

The insulating layer 16A which integrally seals the support body 12 and the component on the support body 12 is made of a polysilazane coat, an insulating resin such as an epoxy resin, a silicone resin, an acrylic resin, a thermoplastic resin, or the like. The process can be simplified by integrally sealing with the insulating layer, in other words, in one process. When these resins are used, it is preferable to use an insulating resin having biocompatibility, high thermal conductivity, and high transmittance with respect to the emission wavelength for the insulating layer 16A. As the insulating resin, polycarbonate (PC), polyethylene terephthalate (PET), polyimide (PI), polyurethane, polyesterimide, or the like whose components and composition are adjusted to reduce adverse effects on the living body can be used. The insulating layer 16A may be formed by providing a biocompatible resin layer on the surface of a polysilazane coat. The insulating layer 16A isolates electrically connected portions between the light-emitting element 11 and the first insulated wire 14a and the second insulated wire 14b from the refrigerant 51. While the sealed state is maintained, the first insulated wire 14a and the second insulated wire 14b are drawn out from the insulating layer 16A in the -Z direction.

The first insulated wire 14a and the second insulated wire 14b are cooled by the refrigerant 51 and also function as heat dissipating materials. The insulated wires 14 are, for example, enameled wires, in each of which a metal wiring of a good conductor such as Cu or Ni is insulated and coated with a polyurethane as an insulating film. Instead of the polyurethane, a coating of an insulating resin such as polyester, polyesterimide, polyamideimide, or polyimide may be applied. By using the insulating film as the surface coating of the insulated wires 14 as a heat dissipating material, heat generated from the light-emitting element 11 can be dissipated to the outside. When a square enameled wire is used as the first insulated wire 14a and the second insulated wire 14b, a wide wiring is formed, and a joint area with the support body 12 can be increased. When the circumscribing circle areas are the same, the square enamel wire can be made smaller in area than the round enamel wire, so that further miniaturization can be achieved.

The light-emitting element 11 is, for example, an end-face light-emitting laser element and resonates in a direction parallel to the Z-axis. When the end-face light-emitting laser element is used as the light-emitting element 11, one end face in the resonance direction is the light-emitting surface 111. The optical component 17 is arranged on the light-emitting element mounting surface 121 of the support body 12 so as to face the light-emitting surface 111 of the light-emitting element 11, and guides light emitted by the end-face light-emitting laser element in a direction that intersects the light-emitting element mounting surface 121. The direction that intersects the light-emitting element mounting surface 121 is all directions except a direction parallel to the light-emitting element mounting surface 121, and can be in a range of, for example, 50° to 130° with respect to the light-emitting element mounting surface 121. The direction may be orthogonal to the light-emitting element mounting surface 121. Herein, the "orthogonal" direction need not be strictly orthogonal to the light-emitting element mounting surface 121, but includes a range of 90° ± 5° in consideration of manufacturing errors and surface conditions of the support body 12 and the optical component 17. Light emitted from the light-emitting element 11 is reflected by the light incident surface 171 of the optical component 17 and irradiated in a predetermined direction. When a polysilazane coat is used for the insulating layer 16A, the light can also be reflected by the polysilazane coat. The optical component 17 may include a combination of a first optical element for controlling the spread angle of light emitted by the light-emitting element 11 and a second optical element having a reflection function as long as a predetermined space 13 is maintained between the light-emitting element 11 and the light-emitting surface 111.

In the configuration example of FIG. 12A, the first insulated wire 14a and the second insulated wire 14b are connected to the upper surface of the light-emitting element 11 and the rear surface 122 of the support body 12, respectively, so as not to interfere with light emission in the Z direction of the light-emitting element 11 and reflection by the optical component 17. A conductive layer 124 is formed at least partially on the front surface of the support body 12. In the example of FIG. 12A, the support body 12 has a light-emitting element mounting surface 121, a rear surface 122, and the conductive layer 124 formed at least partially on the side surface connecting the light-emitting element mounting surface 121 and the rear surface 122. Thus, the first insulated wire 14a is electrically connected directly or indirectly to one electrode of the light-emitting element 11 on the side of the light-emitting element mounting surface 121 of the support body 12. The second insulated wire 14b is electrically connected directly or indirectly to the other electrode of the light-emitting element 11 via the conductive layer 124 formed on the support body 12 on the rear surface 122 of the support body 12.

The configuration of the light-emitting element 11 mounted on the support body 12 of the light irradiation device 10B of FIG. 12B and the insulated wires 14 electrically connected to the light-emitting element 11 is the same as that of the light irradiation device 10A, but are different from FIG. 12A in covering with the insulating layer 16B. The thickness of the insulating layer 16B may be at least partially thicker than that of the light-emitting element 11. In this case, the space between the light-emitting surface 111 of the light-emitting element 11 and the light incident surface 171 of the optical component 17 may be filled with the insulating layer 16B. Light emitted by the light-emitting surface 111 of the light-emitting element 11 passes through the insulating layer 16B and enters the light incident surface 171 of the optical component 17. The material of the insulating layer 16B may be the same as that of the insulating layer 16A described above. By increasing the thickness of the insulating layer 16B, the entirety of the support body 12, the light-emitting element 11, the optical component 17, and the insulated wires 14 can be stably held, and the optical coupling portion and the electrical connection portion can be reliably prevented from coming into contact with the refrigerant. The thickness of the insulating layer 16B may be greater than the thickness of the light-emitting element 11 in the entire region.

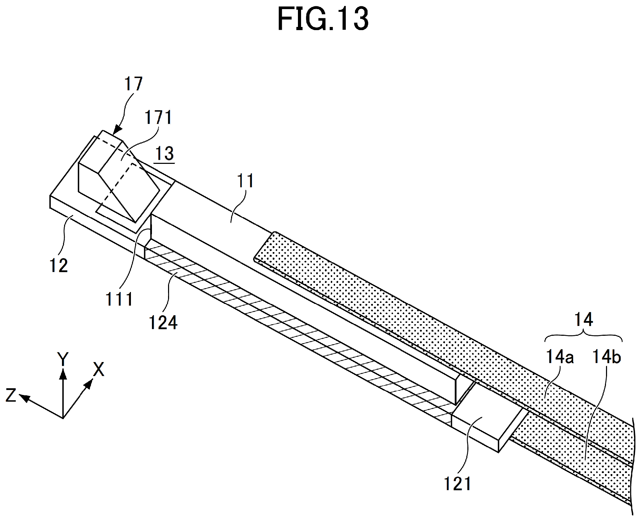

FIG. 13 is a perspective view of a light irradiation device body before being sealed with the insulating layer 16 (or 16A or 16B). A predetermined space 13 is provided between the light-emitting surface 111 of the light-emitting element 11 and the light incident surface 171 of the optical component 17. Light emitted by the light-emitting element 11 enters the optical component 17 through the space 13 and is reflected in a predetermined direction. The conductive layer 124 is formed so as to cover at least a part of the light-emitting element mounting surface 121, at least a part of the side surface, and at least a part of the rear surface 122 of the support body 12. The upper surface of the light-emitting element 11 is connected to the first insulated wire 14a, and the bottom surface of the light-emitting element 11 is connected to the conductive layer 124 formed on the light-emitting element mounting surface 121.

A part of the insulating film of the first insulated wire 14a is removed, and the internal metal wiring is connected to one electrode of the light-emitting element 11. A part of the insulating film of the second insulated wire 14b is removed, and the internal metal wiring is electrically connected to the other electrode of the light-emitting element 11. In the example of FIG. 13, the conductive layer 124 is formed from the light-emitting element mounting surface 121 of the support body 12 through the side surface to the rear surface 122, and the internal metal wiring of the second insulated wire 14b is connected to the conductive layer 124 at the rear surface 122 of the support body 12. Thus, electrical connection is obtained between the other electrode of the light-emitting element 11 and the second insulated wire 14b. By providing the conductive layer 124 for electrical connection on the front surface of the support body 12, the conductive layer 124 can be used as a heat dissipating member.

As described above, the optical component 17 is mounted on the support body 12 and faces the light-emitting surface 111 of the light-emitting element 11. The main body of the optical component 17 is made of a dielectric material such as glass, and a thin silver film is formed on the light incident surface 171, but the present disclosure is not limited to this example. A thin film of aluminum, aluminum alloy, gold, nickel, platinum or the like may be formed on the light incident surface (i.e., reflecting surface) of a main body of an insulating material such as plastic, resin, silicon (Si), aluminum nitride (AlN), silicon nitride (SiN), alumina (Al₂O₃), glass, quartz, or ceramics. Instead of the metal reflecting film, a dielectric multilayer film may be formed. The support body 12, the light-emitting element 11, the optical component 17, and the insulated wires 14 are partially covered with the insulating layer 16A or 16B having light transmissivity by a dip method, a spray method or the like.

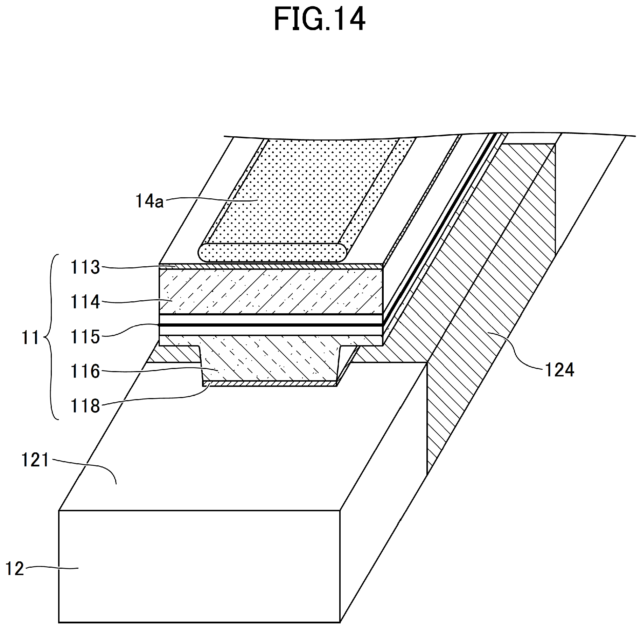

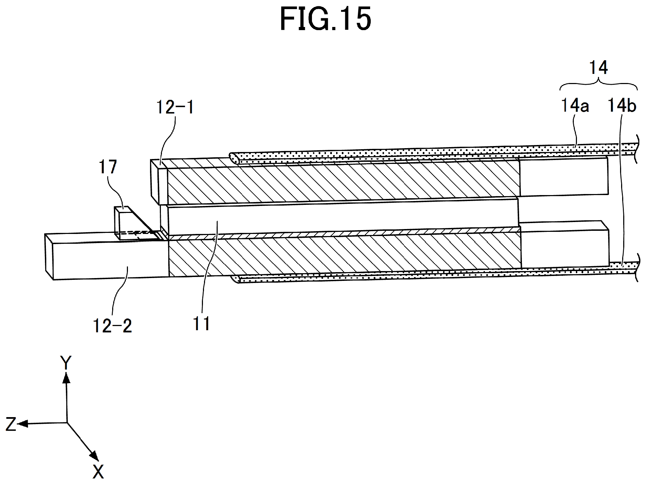

FIG. 14 is a schematic diagram of the light-emitting element 11 mounted on the support body 12. The light-emitting element 11 is, as an example, an edge-emitting laser element having a ridge structure. The light-emitting element 11 has an n-side electrode 113, an n-side semiconductor layer 114, an active layer 115, a p-side semiconductor layer 116, and a p-side electrode 118. The n-side semiconductor layer 114 may include an optical guide layer, a cladding layer, a contact layer, and the like, each doped with an n-type impurity. The p-side semiconductor layer 116 may include an optical guide layer, a cladding layer, a contact layer, and the like, each doped with a p-type impurity. The p-side electrode 118 is electrically connected to the first insulated wire 14a. When an ITO electrode is used as the p-side electrode 118, the p-side electrode 118 may also serve as a cladding layer. The p-side electrode 118 is electrically connected to the second insulated wire 14b via the conductive layer 124 formed on the support body 12. In the example of FIG. 14, the n-side electrode 113 and the first insulated wire 14a are directly connected, but they may be indirectly connected, for example, a second support body may be arranged between the n-side electrode 113 and the first insulated wire 14a. As will be described later with reference to FIG. 15, heat dissipation can be enhanced by arranging the light-emitting element 11 between two supports 12-1 and 12-2.

The semiconductor material and the composition of the light-emitting element 11 are designed to emit laser light of a desired wavelength. When the light-emitting element 11 is used as an ultraviolet laser, a violet laser, a blue laser, and a green laser, GaN-based materials such as GaN, InGaN, and AlGaN are used. When the light-emitting element 11 is used as a red laser, an infrared laser, and a near-infrared laser, GaAs-based materials such as GaAs and AlGaAs, or InP-based materials such as InAlGaP and GaInP are used. In the state where the light-emitting element 11 is mounted on the support body 12, from the viewpoint of sealing with the insulating layer 16A or 16B together with the first insulated wire 14a and the second insulated wire 14b, the width of the ridge may be formed wider to secure the gain of the active layer 115. The width of the ridge may be designed to be, for example, in a range of 2 µm to 100 µm. The transverse mode may be multi-mode or single-mode.

In contrast to an irradiation device configured to irradiate a laser beam through an optical fiber, light emitted by the light-emitting element 11 of the present embodiment is linearly polarized light, and the direction of the linearly polarized light after emission can be changed by changing the reflection direction by the optical component 17 such as a mirror. Thus, for example, it is possible to reduce the reflectance when light enters a living body or to improve the transmittance to the living body. Even when light is in a specific polarization state at the stage when a laser beam is emitted, the polarization state may change or the polarization may be depolarized while passing through a transmission medium such as an optical fiber. In the present embodiment, an affected part in the body can be directly irradiated with light without using an optical fiber, a target part such as an affected part can be irradiated with light while a specific polarization state of the light is maintained.

FIG. 15 is a schematic diagram illustrating an example of a stack of the support body 12 and the light-emitting element 11. In this configuration example, the light-emitting element 11 is arranged between the first support body 12-1 and the second support body 12-2. The first support body 12-1 is in contact with the upper surface of the light-emitting element 11, and the second support body 12-2 is in contact with the bottom surface of the light-emitting element 11. By arranging the light-emitting element 11 between the first support body 12-1 and the second support body 12-2, heat is efficiently dissipated to the upper and lower sides of the light-emitting element 11, and the heat dissipation property of the light irradiation device 10 can be enhanced. The rear end of at least one of the support body 12-1 or the second support body 12-2 may be extended in the -Z direction. Although FIG. 15 assumes that light is extracted in a direction (for example, the Y direction) non-parallel to the optical axis (Z-axis) by the optical component 17, as described in the second embodiment, when light is extracted in a direction parallel to the light-emitting element mounting surface 121 of the support body 12, the front ends of the first support body 12-1 and the second support body 12-2 may be extended in the +Z direction. The insulating layer 16A or 16B covers the entire structure illustrated in FIG. 15.

### (Ninth Embodiment)

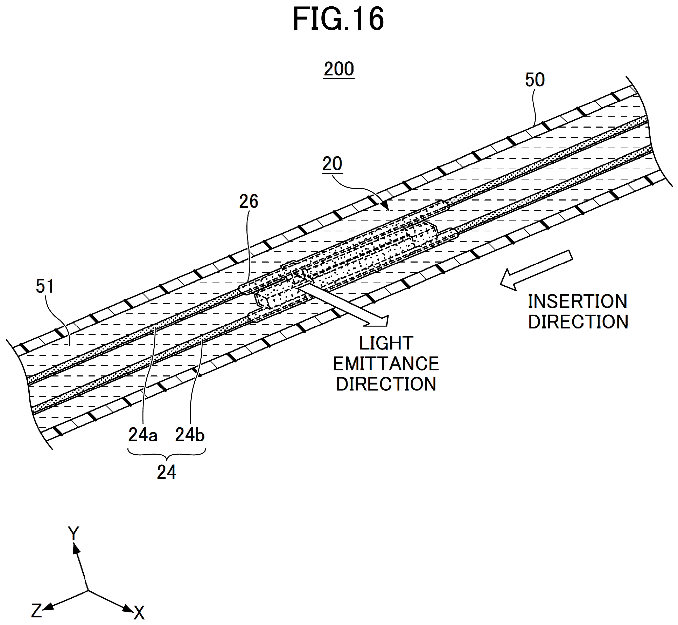

FIG. 16 is a diagram illustrating an example of an in vivo light irradiation assembly 200 using the light irradiation device 20 of the ninth embodiment. The in vivo light irradiation assembly 200 includes the catheter 50 and the light irradiation device 20 inserted into the catheter 50, and the catheter 50 is filled with the refrigerant 51 at least during use. The in vivo light irradiation assembly 200 is used for treatment, diagnosis, sensing, and the like. Similar to the light irradiation device 10 of the eighth embodiment, the light irradiation device 20 is inserted into the catheter 50 in the white arrow labeled as "insertion direction" in the figure and introduced into the body.

The light irradiation device 20 is used in a state in which the heating portion of the light irradiation device 20 is cooled. In the ninth embodiment, an insulated wire 24 is extended from both sides along the optical axis of the insulating layer 26 to enhance heat dissipation. The insulated wire 24 includes a first insulated wire 24a and a second insulated wire 14b, both being electrically connected to the light-emitting element 11. The length of the insulated wire 24 extended from the insulating layer 26 in the +Z direction may be shorter than the length of the insulated wire 24 extended from the insulating layer 26 in the - Z direction. In order to realize this configuration, light emitted by the light-emitting element 11 is guided in a direction parallel to the light-emitting element mounting surface or in a direction not obstructed by the first insulated wire 24a and the second insulated wire 24b. The insulating layer 26 with a small thickness may cover a surface of the support body 12 and the components mounted thereon to conform to surface shapes thereof, as illustrated in FIG. 12A, allowing for enhancing heat dissipation. Alternatively, to ensure electrical insulation from the refrigerant 51, the insulating layer 26 may be locally thickened to fill the spaces between the components while the overall thickness of the light irradiation device is as small as possible.

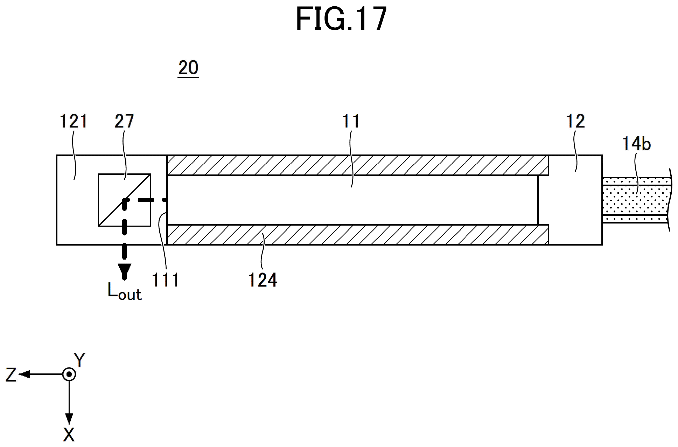

FIG. 17 is a diagram illustrating an arrangement example of an optical component 27 of the light irradiation device 20. For ease of understanding, FIG. 17 illustrates a state before the first insulated wire 24a and the second insulated wire 24b are electrically connected to the light-emitting element 11. Light emitted by the light-emitting surface 111 of the light-emitting element 11 is reflected by the optical component 27 in a direction substantially parallel to the light-emitting element mounting surface 121, and the output light Lₒᵤₜ of the light irradiation device 20 is extracted in the direction indicated by the broken-line arrow in the figure. As long as the reflected light from the optical component 27 is not obstructed by the first insulated wire 24a and the second insulated wire 24b, light may be reflected by the light-emitting element mounting surface 121 in a non-parallel direction.

In the actual light irradiation device 20, the portions where the light-emitting element 11, the optical component 27, and the light-emitting element 11 are electrically connected to the first insulated wire 24a and the second insulated wire 24b are integrally sealed together with the support body 12 by an insulating layer 26. Of the insulating layer 26, at least a portion existing between the light-emitting surface 111 of the light-emitting element 11 and the light incident surface 171 of the optical component is transmissive to light emitted by the light-emitting element 11.

The light-emitting element 11 is, for example, an end surface emitting type laser element. The light-emitting element 11 may be a laser element having a wide ridge structure similar to that of the eighth embodiment. The direction parallel to the Z-axis of the light-emitting element 11 is the resonance direction. The optical component 27 is arranged on the light-emitting element mounting surface 121 of the support body 12, and guides light emitted from the emission surface of the laser element in the direction parallel to the light-emitting element mounting surface 121 of the support body 12. With the first insulated wire 24a and the second insulated wire 24b (see FIG. 16) extended in the +Z direction and the -Z direction with the light-emitting element 11 and the support body 12 located between the first insulated wire 24a and the second insulated wire 24b, light emitted from the light-emitting element 11 is reflected by the optical component 27 in a direction not obstructed by the first insulated wire 24a and the second insulated wire 24b.

The optical component 27 reflects light emitted by the light-emitting surface of the light-emitting element 11, for example, in the X direction parallel to the light-emitting element mounting surface 121. Herein, the "parallel" direction does not need to be strictly parallel to the light-emitting element mounting surface 121, but may have an error of about ±10° including the surface condition of the support body 12 and the manufacturing error of the optical component 27. As long as the laser light reflected by the optical component 27 does not collide with the first insulated wire 24a and the second insulated wire 24b, light may be extracted at an angle other than parallel. The optical component 27 is preferably as thick as the light-emitting element 11 or thinner than the light-emitting element 11. The optical component 27 may be a planar-machined mirror, a prism, or a meta-polarizing element incorporating a meta-surface element. The optical component 27 may also include a lens function for controlling the spread angle of light emitted by the light-emitting element 11. It may be composed of two or more optical elements. For example, the optical component 27 may include one element with a lens function to control the spread angle of the emitted light, and another element to reflect the light, whose spread angle has been controlled, at a predetermined angle.

The insulating layer 26 integrally seals portions where electrical insulation is required, with the first insulated wire 24a and the second insulated wire 24b extending from both ends of the support body 12 in the optical axis direction in the +Z direction and the -Z direction. The electrically connected portions of the light-emitting element 11 and the first insulated wire 24a and the second insulated wire 24b, and at least the light-emitting surface 111 of the light-emitting element 11 and the light incident surface 171 of the optical component 17 are sealed by the insulating layer 26 and isolated from the refrigerant 51. The insulating layer 26 is preferably a biocompatible resin having high thermal conductivity.

At least one of both ends of the support body 12 in the longitudinal direction (Z direction) may protrude from the insulating layer 26. When the support body 12 protruding from the insulating layer 26 comes into contact with the refrigerant 51, heat dissipation of the light irradiation device 20 is ensured. By extending the first insulated wire 24a and the second insulated wire 24b to both sides of the support body 12 in the longitudinal direction, heat dissipation is further enhanced as compared with the eighth embodiment. As in the eighth embodiment, the entire thickness and width of the light irradiation device 20 are about 0.5 mm, and an ultra-compact light irradiation device 20 that can be mounted on the catheter 50 is realized. In the configuration of the ninth embodiment, the light-emitting element 11 may be arranged between two supports 12-1 and 12-2 as illustrated in FIG. 15. In the ninth embodiment, because light emitted by the light-emitting element 11 is extracted in a direction not obstructed by the support body 12, the heat dissipation property of the light irradiation device 20 can be further enhanced by arranging the light-emitting element 11 between the two supports 12-1 and 12-2.

### (Tenth Embodiment)

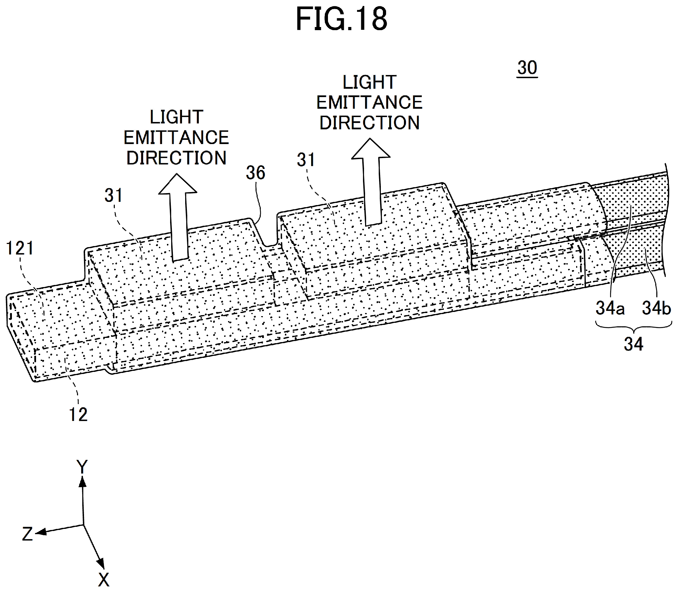

FIG. 18 is a schematic diagram of a light irradiation device 30 of the tenth embodiment. As in the eighth and ninth embodiments, the light irradiation device 30 is also mounted on the catheter 50 (see FIGS. 11 and 16) and used, and the light irradiation device 30 and the catheter 50 can constitute an in vivo light irradiation assembly. In the tenth embodiment, a vertical-cavity surface-emitting laser (VCSEL) is used as a light-emitting element 31.

The light irradiation device 30 includes two light-emitting elements 31 configured to emit light of a predetermined wavelength, the support body 12 on which the light-emitting elements 31 are mounted, and an insulated wire 34 electrically connected to the light-emitting elements 31. The insulated wire 34 includes a first insulated wire 34a connected to one electrode of each of the light-emitting elements 31 and a second insulated wire 34b connected to the other electrode. The light-emitting surface of the light-emitting element 31 and the electrically connected portion between the light-emitting element 31 and the insulated wire 34 are integrally sealed with the support body 12 by an insulating layer 36. At least one of the ends along the longitudinal direction (Z direction) of the support body 12 may protrude from the insulating layer 36. At least a portion of the insulating layer 36 that covers the light-emitting surface is light-transmissive. The thickness of the insulating layer 36 may be smaller or larger than that of the light-emitting element 31.

The light-emitting surface of the VCSEL used for the light-emitting element 31 is parallel to the light-emitting element mounting surface 121 of the support body 12, and a laser light emitted by the VCSEL is emitted in a direction orthogonal to the light-emitting element mounting surface 121 as indicated by the white arrow in the figure. In this configuration, an optical component such as a mirror is not required.

The first insulated wire 34a and the second insulated wire 34b are, for example, 2-core enameled wires. The first insulated wire 34a is connected to one electrode of each light-emitting element 31 on the side of the light-emitting element mounting surface 121 of the support body 12, and the second insulated wire 34b is connected to the other electrode of each light-emitting element 31 on the rear surface of the support body 12 (a surface opposite to the light-emitting element mounting surface 121). The first insulated wire 34a and the second insulated wire 34b are drawn out of the insulating layer 36 from at least one of the ends in the longitudinal direction of the support body 12, with the support body 12 being interposed between the first insulated wire 34a and the second insulated wire 34b.

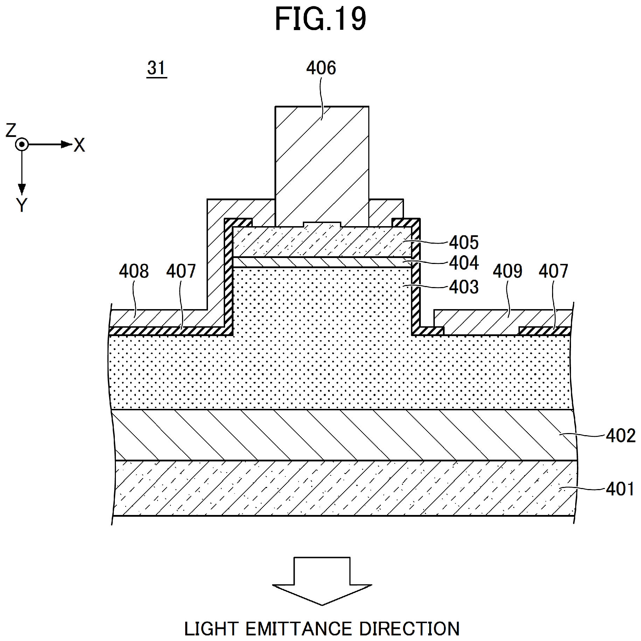

FIG. 19 is a schematic diagram illustrating an example of the light-emitting element 31 used in the tenth embodiment. The VCSEL serving as the light-emitting element 31 has a stacked structure in which a semiconductor substrate 401, an n-side reflecting film 402, an n-type semiconductor layer 403, an active layer 404, a p-type semiconductor layer 405, and a p-side reflecting film 406 are stacked in this order in the -Y direction. The light emission direction is the +Y direction. The p-type and n-type conductivity types may have an inverse relationship. The semiconductor substrate 401 may be removed. The n-type semiconductor layer 403 has a flat plate portion and a protruding portion protruding from the flat plate portion in the -Y direction. The active layer 404 is provided on the upper surface of the convex portion of the n-type semiconductor layer 403. The p-type semiconductor layer 405 is provided on the upper surface of the active layer 404, and the p-side reflecting film 406 is provided on the upper portion of the p-type semiconductor layer 405 except for the peripheral region. A p-side contact layer may be provided between the p-type semiconductor layer 405 and the p-side reflecting film 406.

The light-emitting element 31 is provided with an insulating layer 407 that is provided over the upper surface of the flat plate portion and the side surface of the protruding portion, the side surface of the active layer 404, and the peripheral regions of the side surface and the upper surface of the p-type semiconductor layer 405 in the n-type semiconductor layer 403. The light-emitting element 31 is provided with a p-side electrode 408 electrically connected to the p-type semiconductor layer 405 and an n-side electrode 409 electrically connected to the n-type semiconductor layer 403. The side on which the p-side electrode 408 and the n-side electrode 409 are provided is arranged on the light-emitting element mounting surface 121 of the support body 12. A conductive connection layer (or bump) connected to the light-emitting element mounting surface 212 of the support body 12 may be provided so that the heights of the p-side electrode 408 and the n-side electrode 409 in the -Y direction are aligned. When the light-emitting element 31 is flip-chip mounted on the support body 12 with a conductive connection layer connected to the p-side electrode 408 and the n-side electrode 409, the p-side reflecting film 406 does not come into contact with the light-emitting element mounting surface of the support body 12.

Each of the n-side reflecting film 402 and the p-side reflecting film 406 may be formed of, for example, a DBR (distributed Bragg reflector). The DBR has a structure in which a plurality of high refractive index layers and a plurality of low refractive index layers are alternately stacked. The DBR has a wavelength region of high reflectivity called a stop band. The center wavelength and wavelength width of the stop band are determined by the refractive index and thickness of the high refractive index layer and the refractive index and thickness of the low refractive index layer. The reflectance in the stop band of the DBR increases with the refractive index difference between the high refractive index layer and the low refractive index layer and the number of layers.

In the example illustrated in FIG. 19, a standing wave is formed between the n-side reflecting film 402 and the p-side reflecting film 406. The wavelength in the air of the standing wave is within the stop band of the n-side reflecting film 402 and the p-side reflecting film 406, and this wavelength is the oscillation wavelength of the laser light. An integral multiple of half of the oscillation wavelength is equal to the optical distance between the reflecting surfaces of the n-side reflecting film 402 and the p-side reflecting film 406 facing each other. The optical distance is a distance obtained by multiplying the distance at which light actually propagates through a medium by the refractive index of the medium. By applying a forward voltage between the p-side electrode 408 and the n-side electrode 409, a current can be injected into the active layer 404. The current injection causes an inversion distribution in the active layer 404, and the amplification of light by stimulated emission at the oscillation wavelength, in other words, laser oscillation occurs. As described above, the VCSEL of the present embodiment assumes that the sides of the p-side electrode 408 and the n-side electrode 409 are the mounting surfaces, and the laser light is extracted from the side of the semiconductor substrate 401.

The configuration of the VCSEL illustrated in FIG. 19 is an example. The components included in the VCSEL may be formed of known materials. The shapes of some of the components included in the VCSEL may be changed, and other components may be further provided. Laser light may be extracted from the side opposite to the semiconductor substrate 401.

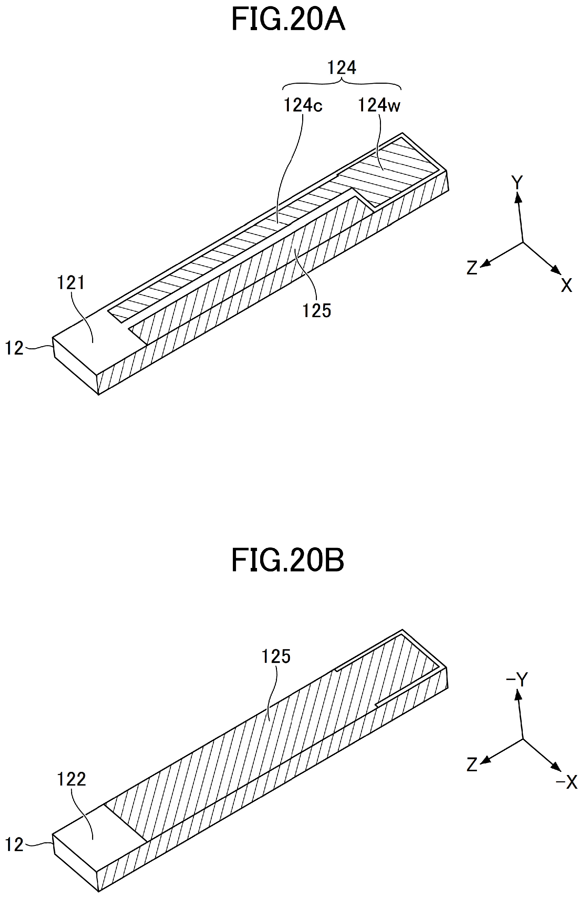

FIG. 20A is a perspective view of the light-emitting element mounting surface 121 side of the support body 12 used in the tenth embodiment, and FIG. 20B is a perspective view of the rear surface 122 side of the support body. Here, the surface opposite to the light-emitting element mounting surface 121 is referred to as the "rear surface". Conductive layers 124 and 125 are formed on the light-emitting element mounting surface 121 of the support body 12. The conductive layer 124 is formed on the light-emitting element mounting surface 121 of the support body 12, and is electrically insulated from the conductive layer 125. The conductive layer 124 has a connection region 124c which is connected to one electrode of the light-emitting element 31, and a wide portion 124w which is wider than the connection region 124c at an end portion on the -Z side of the support body 12. The wide portion 124w is used for electrical connection with the first insulated wire 34a. The conductive layer 125 is formed from the side surface to the rear surface 122 of the support body 12.

The p-side electrode 408 and the n-side electrode 409 of the light-emitting element 31 are connected to the conductive layers 124 and 125 of the light-emitting element mounting surface 121, respectively, via conductive connection layers (or bumps). The wide portion 124w of the conductive layer 124 is electrically connected to the first insulated wire 34a. A part of the insulating film of the first insulated wire 34a is removed, and the internal metal wiring is connected to the conductive layer 124. The conductive layer 125 is electrically connected to the second insulated wire 34b at the rear surface 122 of the support body 12. A part of the insulating film of the second insulated wire 34b is removed, and the internal metal wiring is connected to the conductive layer 125.

The first insulated wire 34a and the second insulated wire 34b extend in the longitudinal direction (Z direction) of the support body 12, with the support body being interposed between the first insulated wire 34a and the second insulated wire 34b, and do not hinder light emission from the VCSEL. In the arrangement illustrated in FIG. 18, the second insulated wire 34b connected to the rear surface 122 of the support body 12 may be extended to the vicinity of the tip of the support body 12 on the +Z side. The first insulated wire 34a and the second insulated wire 34b protruding from the insulating layer 36 in the -Z direction are brought into contact with the refrigerant and cooled. The support body 12 may also protrude from the insulating layer 36 at at least one end in the longitudinal direction and be directly cooled by the refrigerant. The conductive layers 124 and 125 formed on the surface of the support body 12 also function as a heat sink.

The VCSEL chip used as the light-emitting element 31 has a side length and a height of 200 µm or less, and is mounted on the support body 12 and sealed with the insulating layer 36. The overall thickness and width of the light irradiation device 30 are about 0.5 mm, and an ultra-compact light irradiation device 30 mountable on the catheter 50 is realized. In the example of FIG. 18, the thickness of the insulating layer 36 is smaller than the thickness of the light-emitting element 31.

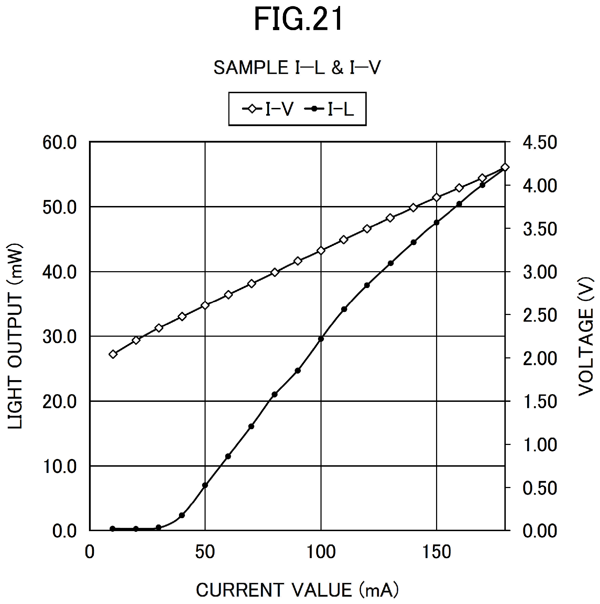

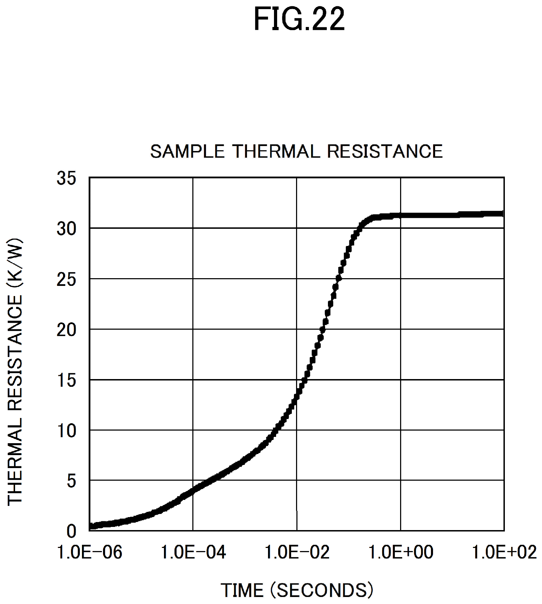

FIG. 21 is a diagram illustrating electrical characteristics and optical output characteristics of the fabricated light irradiation device, and FIG. 22 is a diagram illustrating thermal resistance. The characteristics of the light irradiation device were measured by fabricating a sample of the configuration of the eighth embodiment. The used light-emitting element 11 has a cavity length (length in the Z direction) of 1.5 mm, a width (length in the X direction) of 0.2 mm, and an oscillation wavelength of 640 nm. The support body 12 is an AlN substrate having a thickness (length in the Y direction) of 0.1 mm. The mirror is a silver mirror, and the size (length × width) of the bottom surface is 0.3 mm × 0.2 mm, and the height is 0.2 mm. The first insulated wire 14a and the second insulated wire 14b are polyurethane copper wires having a diameter of 0.1 mm and a length of 1.5 m. The insulating layer 16 is a polysilazane coat.

In FIG. 21, the horizontal axis indicates the current value [mA] applied through the insulated wire 14, the vertical axis on the left indicates the light output [mW], and the vertical axis on the right indicates the voltage [V]. In the figure, the black dots indicate the current versus the light output characteristic, and the white dots indicates the current versus the voltage characteristics. The optical output increases as the current increases, and an optical power of 30 mW is obtained at an injection current of 100 mA.

In FIG. 22, the horizontal axis represents time [seconds], and the vertical axis represents thermal resistance [K/W]. The thermal resistance is saturated in about 0.1 seconds after the operation of the light irradiation device is turned on. By cooling the light irradiation device with the refrigerant 51 during operation, heat can be efficiently released from the support body 12, the insulated wire 14, and the insulating layer 16, thereby stabilizing the operation of the light irradiation device.

Although the above description has been based on a specific configuration example, the present disclosure is not limited to the above-described configuration example. For example, in the eighth embodiment, the second insulated wire 14b, which does not hinder light emission, may be extended in the +Z direction from the tip of the support body 12 to function as a heat dissipation member. In the configuration of the eighth embodiment or the tenth embodiment, a light-emitting element and a photodetector such as a photodiode may be arranged on the light-emitting element mounting surface 121 of the support body 12 to be used as a biological sensor. In the configuration of the tenth embodiment, instead of mounting two light-emitting elements 31 on the support body 12, one light-emitting element 31 and one light-receiving element may be mounted. When 2-core enameled wires are used as the insulated wires 14 and 34, one metal wire may be used for supplying an electric signal to the light-emitting element 11 or 31, and the other metal wire may be used as a read wire for a signal that is output from a photodetector.

The light irradiation devices 10 (including 10A and 10B), 20, and 30 of the eighth through tenth embodiments may be used in combination with an endoscope. The light-emitting elements 11 and 31 can be used not only as a laser light source for treatment but also as a sensing light source or an illumination light source. In either case, the light irradiation device has a heat dissipation property capable of dissipating heat from the light-emitting element to the outside and a sufficient electrical insulation property to ensure insulation in a refrigerant, and exhibits an effect as a light irradiation device mountable on a catheter. Unlike an optical fiber, an insulated wire having an insulating film has a strong flexibility. A configuration in which the light-emitting element itself is mounted on the support body 12 is excellent in integration, and the range of application to a sensor is wide.

The present disclosure is applicable not only to a light irradiation device inserted into a medical catheter, but also to other medical applications. It is also applicable to applications such as a sensor requiring local light irradiation in combination with a refrigerant.

The light irradiation devices of the first through seventh embodiments are not limited to those arranged inside a living body for medical use, but can also be used as an illumination light source arranged in a narrow space inside a precision machine or the like and used for inspecting the inside of a precision machine or the like.

### (Eleventh Embodiment )

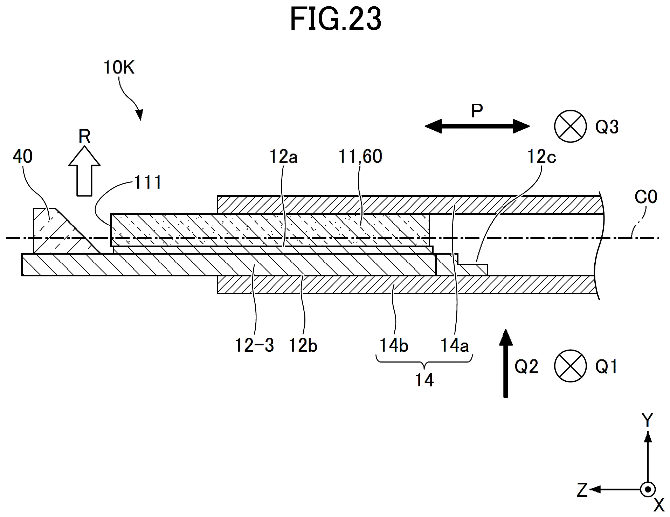

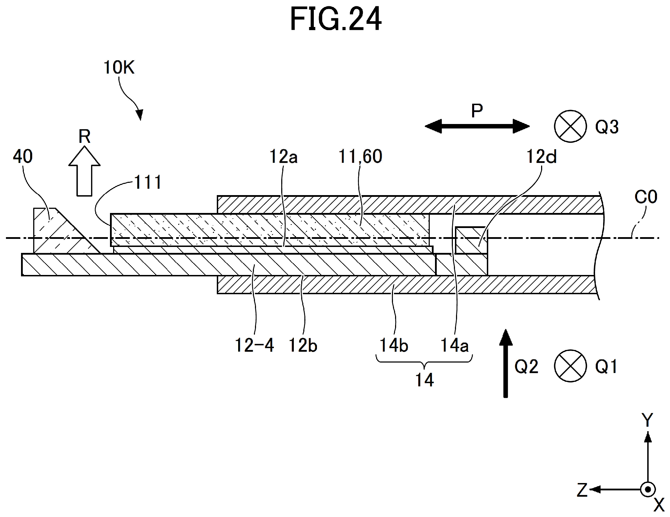

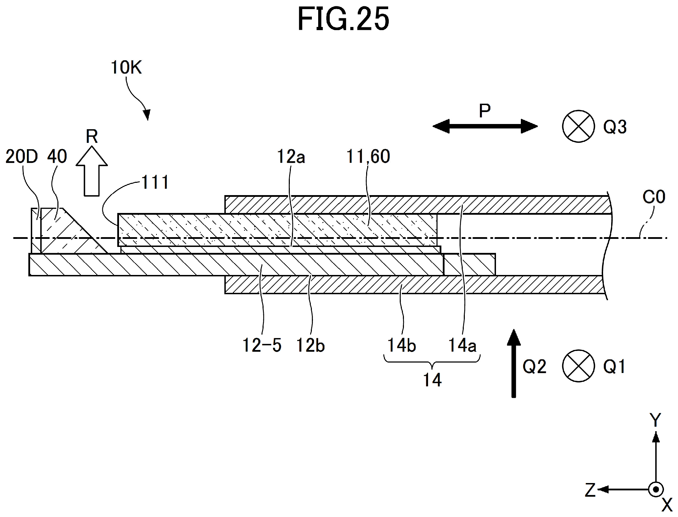

FIG. 23 is a schematic cross-sectional view illustrating a first example of a configuration of a light irradiation device 10K of the eleventh embodiment. FIG. 24 is a schematic cross-sectional view illustrating a second example of the configuration of the light irradiation device 10K of the eleventh embodiment. FIG. 25 is a schematic cross-sectional view illustrating a third example of the configuration of the light irradiation device 10K of the eleventh embodiment. FIGS. 23 through 25 illustrate cross-sections including a central axis C0 along the longitudinal direction P in which the light irradiation device 10K extends.

Similar to the eighth through tenth embodiments, the light irradiation device 10K may also be mounted and used in the catheter 50 (see FIGS. 11 and 16), and the light irradiation device 10K and the catheter 50 may constitute an in vivo light irradiation assembly. In the eleventh embodiment, the light-emitting part 60 includes the light-emitting element 11 and the support body 12-3 for supporting the light-emitting element 11. The support body 12-3 includes a member having radiopacity to radiation such as X-rays. For example, the support body 12-3 includes Cu-W or Cu-Mo. Because the support body 12-3 includes a member having radiopacity to radiation such as X-rays, it is not necessary to provide a marker member separately. The support body 12-3 has a notch 12c on the opposite side of the optical component 40 across the light-emitting part 60. The support body 12-3 can also serve as a marker member or a marker, allowing miniaturization of the light irradiation device 10K. The light-emitting element 11 is fixed to the support body 12-3 and the support body 12-3 and the light-emitting surface 111 are arranged close to each other, which can enhance the accuracy of the direction of light irradiation. The support body 12-3 may be a block made of Cu-W or Cu-Mo. When the support body 12-3 has conductivity, the support body 12-3 may also serve as a conductive layer.

The light-emitting element 11 is arranged on a first surface 12a, which is the upper surface of the support body 12-3. The light-emitting element 11 can emit light in a direction along the longitudinal direction P from the light-emitting surface 111 that intersects the longitudinal direction P. In the example illustrated in FIG. 23, the light-emitting element 11 emits light in the +Z direction. Because the light-emitting part 60 of the light irradiation device 10K includes the light-emitting element 11 and the support body 12-3, the support body 12-3 can be used as a heat dissipating member for dissipating heat of the light-emitting element 11.

The support body 12-3 has radiopacity to radiation such as X-rays and has a notch 12c, and differs in at least one of shape or position when viewed in the first direction Q1 and when viewed in the second direction Q2. Therefore, when a living body in which the light irradiation device 10K is arranged is imaged by X-ray CT or the like, the image of the support body 12-3 can be confirmed in the captured image. By confirming at least one of shape or position of the image of the support body 12-3 reflected in the captured image by X-ray CT, the orientation of the support body 12 arranged in the living body can be determined. The orientation of the support body 12-3 is known with respect to the longitudinal direction P, and the light irradiation device 10K can direct light emitted by the light-emitting element 11 toward the predetermined direction R that intersects the longitudinal direction P. Therefore, in the present embodiment, in using the light irradiation device 10K, the irradiation direction of light from the light irradiation device 10K can be determined from the orientation of the support body 12-3 ascertained on the basis of the captured image by X-ray CT.

In the first example illustrated in FIG. 23, the support body 12-3 has a notch 12c, but as in the second example illustrated in FIG. 24, a support body 12-4 may have a protruding portion 12d protruding in the +Y direction from the first surface 12a, which is the upper surface of the support body 12-4. The support body 12-4 including the protruding portion 12d may be formed as an integral member or may be formed by joining rectangular parallelepiped shaped members of different sizes. The support body 12-4, similarly to the support body 12-3, has radiopacity to radiation such as X-rays.

Alternatively, as in the third example illustrated in FIG. 25, a support body 12-5 and the marker 20D provided on the optical component 40 may be used together. The support body 12-5, like the support body 12-3, has radiopacity to radiation such as X-rays.

This application claims priority based on Japanese Patent Application No. 2023-217446, filed with the Japan Patent Office on December 22, 2023, and Japanese Patent Application No. 2023-217447, filed with the Japan Patent Office on December 22, 2023, and includes the entire contents of these Japanese Patent Applications.

Embodiments of the present disclosure can include following configurations:
<Aspect 1> A light irradiation device extending in a longitudinal direction, the light irradiation device comprising:
   a light-emitting part;
   a marker member directly or indirectly connected to the light-emitting part and having radiopacity, wherein
   the marker member differs in at least one of shape or position when viewed in a first direction orthogonal to the longitudinal direction and when viewed in a second direction different from the first direction and orthogonal to the longitudinal direction, and
   the light irradiation device is configured to irradiate light from the light-emitting part in a predetermined direction that intersects the longitudinal direction.
<Aspect 2> The light irradiation device according to <Aspect 1>, wherein
   the marker member includes
   an elongated base; and
   at least one of a protrusion or a recess provided on a surface of the base.
<Aspect 3> The light irradiation device according to <Aspect 1> or <Aspect 2>, the light irradiation device further comprising:
   an optical component configured to reflect light emitted by the light-emitting part in a direction along the longitudinal direction in a direction that intersects the longitudinal direction.
<Aspect 4> The light irradiation device according to any one of <Aspect 1> to <Aspect 3>, wherein
   the light-emitting part includes
   a light-emitting element; and
   a support body supporting the light-emitting element.
<Aspect 5> The light irradiation device according to <Aspect 4>, further comprising an insulating layer, wherein
   the insulating layer is provided over at least one of the light-emitting element or the support body.
<Aspect 6> The light irradiation device according to <Aspect 4> or <Aspect 5>, further comprising:
   an insulated wire electrically connected to the light-emitting element, wherein
   the insulated wire is electrically insulated from the marker member.
<Aspect 7> The light irradiation device according to any one of <Aspect 4> to <Aspect 6>, further comprising:
   a casing including an opening and configured to accommodate at least a part of the light-emitting element and at least a part of the support body; and
   a light-transmissive member configured to seal the opening of the casing, wherein
   the light-transmissive member transmits light emitted from the light-emitting part and causes the light to travel in the predetermined direction that intersects the longitudinal direction.
<Aspect 8> The light irradiation device according to <Aspect 7>, wherein
   the light-emitting element is arranged inside the casing, and
   the inside of the casing is hermetically sealed.
<Aspect 9> A light irradiation device extending in a longitudinal direction, the light irradiation device comprising:
   a light-emitting part;
      a support body to which the light-emitting part is fixed; and
      a marker having radiopacity, wherein
   the marker differs in at least one of shape or position between when viewed in a first direction orthogonal to the longitudinal direction and when viewed in a second direction different from the first direction and orthogonal to the longitudinal direction,
   the marker is provided on the support body, and
   the light irradiation device is configured to irradiate light from the light-emitting part in a predetermined direction that intersects the longitudinal direction.
<Aspect 10> The light irradiation device according to <Aspect 9>, wherein
   the support body includes:
      a first surface on which the light-emitting part is arranged;
         and
      a second surface located on a side opposite to the first surface, wherein
   the marker is provided on the second surface of the support body.
<Aspect 11> The light irradiation device according to <Aspect 9> or <Aspect 10>, wherein
   a thickness of at least a part of the marker is equal to or greater than 20 µm and equal to or less than 100 µm.
<Aspect 12> The light irradiation device according to any one of <Aspect 9> to <Aspect 11>, wherein
   the support body includes:
      a first surface on which the light-emitting part is arranged;
         and
      a second surface located on the side opposite to the first surface, wherein
   a position of the marker when viewed in a third direction, which is parallel to the first surface, differs from a position of the marker when viewed in the third direction in a state in which the light irradiation device is rotated by 180 degrees with a central axis of the light irradiation device parallel to the longitudinal direction serving as a rotation axis, by at least twice a distance between the first surface and the second surface of the support body.
<Aspect 13> The light irradiation device according to any one of <Aspect 9> to <Aspect 12>, wherein
   the support body includes:
      a first surface on which the light-emitting part is arranged;
         and
      a second surface located on a side opposite to the first surface,
   the marker is provided on each of the first surface and the second surface, and
   the marker provided on the first surface differs from the marker provided on the second surface in at least one of size or position of the marker.
<Aspect 14> The light irradiation device according to any one of <Aspect 9> to <Aspect 13>, wherein
   the light-emitting part is a light-emitting element, and
   the light-emitting element is arranged on the support body.
<Aspect 15> The light irradiation device according to any 1 of <Aspect 9> to <Aspect 14>, wherein
   the light-emitting part is a fiber,
   the fiber is arranged on the support body.
<Aspect 16> The light irradiation device according to <Aspect 14>, wherein
   the support body includes:
      a first surface on which the light-emitting part is arranged;
         and
      a second surface located on a side opposite to the first surface, wherein
   the second surface of the support body includes a first region and a second region different from the first region,
   the marker is provided in the first region, and
   an insulated wire is arranged in the second region.
<Aspect 17> A light irradiation device extending in a longitudinal direction, the light irradiation device comprising:
   a light-emitting part;
   a support body to which the light-emitting part is fixed;
   an optical component that has an optical effect of at least one of reflection, refraction or diffraction with respect to light emitted by the light-emitting part; and
   a marker having radiopacity, wherein
   the marker differs in at least one of shape or position between when viewed in a first direction orthogonal to the longitudinal direction and when viewed in a second direction different from the first direction and orthogonal to the longitudinal direction,
   the marker is provided in the optical component, and
   the light irradiation device is configured to irradiate light from the light-emitting part in a predetermined direction that intersects the longitudinal direction.
<Aspect 18> The light irradiation device according to <Aspect 17>, wherein
   the light-emitting part is configured to emit the light in a direction along the longitudinal direction,
   the optical component includes a reflecting surface for reflecting the light from the light-emitting part in a direction that intersects the longitudinal direction, and
   the marker is provided in a region of the optical component other than a region provided with the reflecting surface.
<Aspect 19> The light irradiation device according to <Aspect 17>, further comprising:
   a second marker provided in a portion other than the support body and the optical component.
<Aspect 20> The light irradiation device according to any one of <Aspect 17> to <Aspect 19>, wherein
   the support body and the optical component are integrally formed.
<Aspect 21> A light irradiation device extending in a longitudinal direction, the light irradiation device comprising:
   a light-emitting part comprising
      a light-emitting element, and
      a support body for supporting the light-emitting element,
         wherein
   the support body includes a component having radiopacity,
   the support body differs in at least one of shape or position when viewed in a first direction orthogonal to the longitudinal direction and when viewed in a second direction different from the first direction and orthogonal to the longitudinal direction, and
   the light irradiation device is configured to irradiate light from the light-emitting part in a predetermined direction that intersects the longitudinal direction.

### REFERENCE CHARACTERS LIST

10, 10A, 10B, 10C, 10D, 10E, 10F, 10G, 10H, 10J, 10K, 20, 30
Light irradiation device
11, 31 Light-emitting element
111 Light-emitting surface
12, 12-1, 12-2, 12-3, 12-4 Support
12a First surface
12b Second surface
12b1 First region
12b2 Second region
12c Notch
12d Protruding portion
121 Light-emitting element mounting surface
122 Rear surface
124, 125 Conductive layer
14, 24, 34 Insulated wire
14a, 24a, 34a First insulated wire
14b, 24b, 34b Second insulated wire
16, 16A, 16B, 26, 36 Insulating layer
17, 27 Optical component
18 Fiber
20A Marker member
20D Marker
20J Second marker
20v First position
20u Second position
t1 thickness
t2, d Distance
30A Casing
31A Opening
32 Light-transmissive member
40 Optical component
41 Reflecting surface
42 Surface
50 Catheter
51 Refrigerant
60 Light-emitting part
100, 200 In vivo light irradiation assembly
Q1 First direction
Q2 Second direction
Q3 Third direction
P Longitudinal direction
R Direction

## Claims

1. A light irradiation device extending in a longitudinal direction, the light irradiation device comprising:
a light-emitting part;
a marker member directly or indirectly connected to the light-emitting part and having radiopacity, wherein
the marker member differs in at least one of shape or position when viewed in a first direction orthogonal to the longitudinal direction and when viewed in a second direction different from the first direction and orthogonal to the longitudinal direction, and
the light irradiation device is configured to irradiate light from the light-emitting part in a predetermined direction that intersects the longitudinal direction.

2. The light irradiation device according to claim 1, wherein
the marker member includes
an elongated base; and
at least one of a protrusion or a recess provided on a surface of the base.

3. The light irradiation device according to claim 1 or claim 2, the light irradiation device further comprising:
an optical component configured to reflect light emitted by the light-emitting part in a direction along the longitudinal direction in a direction that intersects the longitudinal direction.

4. The light irradiation device according to any one of claims 1 to 3, wherein
the light-emitting part includes
a light-emitting element; and
a support body supporting the light-emitting element.

5. The light irradiation device according to claim 4, further comprising an insulating layer, wherein
the insulating layer is provided over at least one of the light-emitting element or the support body.

6. The light irradiation device according to claim 4 or claim 5, further comprising:
an insulated wire electrically connected to the light-emitting element, wherein
the insulated wire is electrically insulated from the marker member.

7. The light irradiation device according to any one of claims 4 to 6, further comprising:
a casing including an opening and configured to accommodate at least a part of the light-emitting element and at least a part of the support body; and
a light-transmissive member configured to seal the opening of the casing, wherein
the light-transmissive member transmits light emitted from the light-emitting part and causes the light to travel in the predetermined direction that intersects the longitudinal direction.

8. The light irradiation device according to claim 7, wherein
the light-emitting element is arranged inside the casing, and
the inside of the casing is hermetically sealed.

9. A light irradiation device extending in a longitudinal direction, the light irradiation device comprising:
a light-emitting part;
a support body to which the light-emitting part is fixed; and
a marker having radiopacity, wherein
the marker differs in at least one of shape or position between when viewed in a first direction orthogonal to the longitudinal direction and when viewed in a second direction different from the first direction and orthogonal to the longitudinal direction,
the marker is provided on the support body, and
the light irradiation device is configured to irradiate light from the light-emitting part in a predetermined direction that intersects the longitudinal direction.

10. The light irradiation device according to claim 9, wherein
the support body includes:
a first surface on which the light-emitting part is arranged; and
a second surface located on a side opposite to the first surface, wherein
the marker is provided on the second surface of the support body.

11. The light irradiation device according to claim 9 or claim 10,
wherein
a thickness of at least a part of the marker is equal to or greater than 20 µm and equal to or less than 100 µm.

12. The light irradiation device according to any one of claims 9 to 11,
wherein
the support body includes:
a first surface on which the light-emitting part is arranged; and
a second surface located on the side opposite to the first surface, wherein
a position of the marker when viewed in a third direction, which is parallel to the first surface, differs from a position of the marker when viewed in the third direction in a state in which the light irradiation device is rotated by 180 degrees with a central axis of the light irradiation device parallel to the longitudinal direction serving as a rotation axis, by at least twice a distance between the first surface and the second surface of the support body.

13. The light irradiation device according to any one of claims 9 to 12, wherein
the support body includes:
a first surface on which the light-emitting part is arranged; and
a second surface located on a side opposite to the first surface,
the marker is provided on each of the first surface and the second surface, and
the marker provided on the first surface differs from the marker provided on the second surface in at least one of size or position of the marker.

14. The light irradiation device according to any one of claims 9 to 13, wherein
the light-emitting part is a light-emitting element, and
the light-emitting element is arranged on the support body.

15. The light irradiation device according to any 1 of claims 9 to 14, wherein
the light-emitting part is a fiber,
the fiber is arranged on the support body.

16. The light irradiation device according to claim 14, wherein
the support body includes:
a first surface on which the light-emitting part is arranged; and
a second surface located on a side opposite to the first surface, wherein
the second surface of the support body includes a first region and a second region different from the first region,
the marker is provided in the first region, and
an insulated wire is arranged in the second region.

17. A light irradiation device extending in a longitudinal direction,
the light irradiation device comprising:
a light-emitting part;
a support body to which the light-emitting part is fixed;
an optical component that has an optical effect of at least one of reflection, refraction or diffraction with respect to light emitted by the light-emitting part; and
a marker having radiopacity, wherein
the marker differs in at least one of shape or position between when viewed in a first direction orthogonal to the longitudinal direction and when viewed in a second direction different from the first direction and orthogonal to the longitudinal direction,
the marker is provided in the optical component, and
the light irradiation device is configured to irradiate light from the light-emitting part in a predetermined direction that intersects the longitudinal direction.

18. The light irradiation device according to claim 17, wherein
the light-emitting part is configured to emit the light in a direction along the longitudinal direction,
the optical component includes a reflecting surface for reflecting the light from the light-emitting part in a direction that intersects the longitudinal direction, and
the marker is provided in a region of the optical component other than a region provided with the reflecting surface.

19. The light irradiation device according to claim 17, further comprising:
a second marker provided in a portion other than the support body and the optical component.

20. The light irradiation device according to any one of claims 17 to 19, wherein
the support body and the optical component are integrally formed.

21. A light irradiation device extending in a longitudinal direction, the light irradiation device comprising:
a light-emitting part comprising
a light-emitting element, and
a support body for supporting the light-emitting element, wherein
the support body includes a component having radiopacity,
the support body differs in at least one of shape or position when viewed in a first direction orthogonal to the longitudinal direction and when viewed in a second direction different from the first direction and orthogonal to the longitudinal direction, and
the light irradiation device is configured to irradiate light from the light-emitting part in a predetermined direction that intersects the longitudinal direction.
